# EUROPEAN PATENT SPECIFICATION

(11) **EP 4 447 863 B1**
(45) Date of publication and mention of the grant of the patent: **17.12.2025**
(21) Application number: 22797194.2
(22) Date of filing: 04.10.2022
(51) Int. Cl.: A61F 2/24

(54) **APPARATUS FOR MONITORING RADIAL EXPANSION OF PROSTHETIC DEVICE**
VORRICHTUNG ZUR ÜBERWACHUNG DER RADIALEN EXPANSION EINER PROTHESENVORRICHTUNG
APPAREIL DE SURVEILLANCE D'EXPANSION RADIALE D'UN DISPOSITIF PROTHÉTIQ

(30) Priority: 17.12.2021 US 202163291172 P
(43) Date of publication of application: 23.10.2024
(73) Proprietor: Edwards Lifesciences Corporation, Irvine, CA 92614 (US)
(72) Inventor: DVORSKY, Anatoly, 3079892 Caesarea (IL); GROSU, Eran, 3079892 Caesarea (IL); BUKIN, Michael, 3079892 Caesarea (IL); GUROVICH, Nikolay, 3079892 Caesarea (IL)
(74) Representative: Eisenführ Speiser
(86) International application number: PCT/US2022/045636
(87) International publication number: WO 2023/113904

(56) References cited:
- US-A1- 2013 046 373
- US-A1- 2019 060 071
- US-A1- 2021 275 299
- US-A1- 2021 307 904

## Description

### CROSS-REFERENCE TO RELATED APPLICATION

This application claims the benefit of U.S. Provisional Application No. 63/291,172, filed December 17, 2021.

### FIELD

The present disclosure relates to implantable, radially expandable prosthetic devices, such as prosthetic heart valves, and to methods, assemblies, and apparatuses for delivering, expanding, implanting, and deploying such prosthetic devices.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (e.g., through a femoral artery and the aorta) until the prosthetic heart valve reaches the implantation site in the heart. The prosthetic heart valve is then expanded to its functional size, for example, by inflating a balloon on which the prosthetic valve is mounted, actuating a mechanical actuator that applies an expansion force to the prosthetic heart valve, or by deploying the prosthetic heart valve from a sheath of the delivery apparatus so that the prosthetic heart valve can self-expand to its functional size.

US 2013/046373 A1 relates to sealable and repositionable implant devices provided with features that increase the ability of implants such as endovascular grafts and valves to be precisely deployed or re-deployed, with better in situ accommodation to the local anatomy of the targeted recipient anatomic site, and/or with the ability for post-deployment adjustment to accommodate anatomic changes that might compromise the efficacy of the implant. Insofar, a surgical implant is suggested which includes an implant body and a selectively adjustable assembly attached to the implant body, the assembly having adjustable elements and being operable to cause a configuration change in a portion of the implant body and, thereby, permit implantation of the implant body within an anatomic orifice to effect a seal therein under normal physiological conditions.

US 2021/275299 A1 relates to a transcatheter valve prosthesis including a stent having a crimped configuration for delivery within a vasculature and an expanded configuration for deployment within a native heart valve. In this arrangement, the stent includes at least one axial frame member and the transcatheter valve prosthesis includes at least one outflow marker positioned on the at least one axial frame member. The transcatheter heart valve prosthesis may include inflow markers positioned within the inflow portion of the stent and two outflow markers disposed on axial frame members and being circumferentially and longitudinally offset from each other. The markers are configured to aid in axial and circumferential alignment of the transcatheter heart valve prosthesis within a native heart valve.

Prosthetic heart valves that rely on a mechanical actuator for expansion can be referred to as "mechanically expandable" prosthetic heart valves. Mechanically expandable prosthetic heart valves can provide one or more advantages over self-expandable and balloon-expandable prosthetic heart valves. For example, mechanically expandable prosthetic heart valves can be expanded to various fully functional working diameters. Some mechanically expandable prosthetic heart valves can also be compressed after an initial expansion (e.g., for repositioning and/or retrieval).

Despite the recent advancements in percutaneous valve technology, there remains a need for improved transcatheter prosthetic devices. For example, there is room for improvement for expanding mechanically expandable prosthetic devices evenly and monitoring radial expansion of the prosthetic devices in real-time.

### SUMMARY

The present disclosure relates to methods and devices for treating valvular diseases. Specifically, the present disclosure is directed to implantable, radially expandable prosthetic devices, such as prosthetic heart valves, and to methods, assemblies, and apparatuses for delivering, expanding, implanting, and deploying such prosthetic devices. More specifically, the present disclosure describes techniques that allow an operator to monitor radial expansion of a prosthetic device under fluoroscopy.

In an aspect, a prosthetic device can comprise a radially compressible and expandable frame. In addition to these components, a prosthetic device can further comprise one or more of the components disclosed herein.

In some examples, the prosthetic device can be a prosthetic heart valve that includes a valvular structure (for example, one or more leaflets) disposed in the frame.

In some examples, a prosthetic device can comprise two or more pairs of first movable markers and second movable markers that are circumferentially spaced apart from each other.

In some examples, the first and second movable markers of each pair are axially aligned and axially movable relative to one another as the frame radially expands such that an axial distance between the first and second movable markers of each pair can change.

In some examples, positions of the first and second movable markers are configured so that a difference between an axial distance of one of the pairs of first and second movable markers and an axial distance of another one of the pairs of first and second movable markers can indicate a degree of evenness of the frame's radial expansion.

In some examples, a prosthetic device can comprise one or more screw actuators coupled to the frame and configured to be rotated to radially expand the frame from a radially compressed state to a radially expanded state.

In some examples, the frame can include a pair of first and second fixed markers that are axially spaced apart from one another by a fixed distance and a pair of first and second movable markers that are axially movable relative to one another as the frame radially expands so that a distance between the pair of first and second movable markers can be compared to the fixed distance between the pair of first and second fixed markers.

Certain aspects of the disclosure concern a prosthetic device that includes a radially compressible and expandable frame and a screw actuator coupled to the frame and configured to be rotated to radially expand the frame from a radially compressed state to a radially expanded state. The frame can include two or more pairs of first movable markers and second movable markers. The pairs of first and second movable markers can be circumferentially spaced apart from each other. The first and second movable markers of each pair can be axially aligned and axially movable relative to one another as the frame radially expands Such that an axial distance between the first and second movable markers of each pair can change. Positions of the first and second movable markers can be configured so that a difference between an axial distance of one of the pairs of first and second movable markers and an axial distance of another one of the pairs of first and second movable markers can indicate a degree of evenness of the frame's radial expansion.

Certain aspects of the disclosure concern another prosthetic device including a radially compressible and expandable frame and a screw actuator coupled to the frame and configured to be rotated to radially expand the frame from a radially compressed state to a radially expanded state. The frame can include a pair of first and second fixed markers that are axially spaced apart from one another by a fixed distance and a pair of first and second movable markers that are axially movable relative to one another as the frame radially expands so that a distance between the pair of first and second movable markers can be compared to the fixed distance between the pair of first and second fixed markers.

According to certain aspects of the disclosure, a prosthetic device can include a frame, which can include a plurality of pairs of axially extending first and second actuator posts, at least one rod extending through an inner bore of a selected second actuator post and coupled to a selected first actuator post paired with the selected second actuator post, and two or more pairs of first movable markers and second movable markers. Each pair of the first and second actuator posts can be axially movable relative to one another. Rotation of the rod can be configured to radially expand the frame from a radially compressed state to a radially expanded state. The first movable markers can be located on two or more first actuator posts and the second movable markers can be located on two or more second actuator posts paired with the two or more first actuator posts. The first and second movable markers of each pair are spaced from each other by an axial distance that decreases as the frame is radially expanded. Positions of the first and second movable markers can be configured so that a difference between an axial distance of one of the pairs of first and second movable markers and an axial distance of another one of the pairs of first and second movable markers as the frame is radially expanded can indicate a degree of evenness of the frame's radial expansion.

According to certain aspects of the disclosure, a prosthetic device can include a frame which includes axially extending first and second actuator posts that are axially movable relative to one another, a rod extending through an inner bore of the second actuator post and coupled to the first actuator post, and a support post extending in parallel to the first and second actuator posts. The rod can be configured to be rotated to radially expand the frame from a radially compressed state to a radially expanded state. The support post can include a pair of first and second fixed markers that are axially spaced apart from one another by a fixed distance. The first actuator post can include a first movable marker and the second actuator post can include a second movable marker so that a distance between the first and second movable markers can be compared to the fixed distance between the first and second fixed markers.

Certain aspects of the disclosure also concern a prosthetic valve. The prosthetic valve can include an annular frame having an inflow end and an outflow end, and a valvular structure mounted within the frame and configured to regulate blood flow from the inflow end to the outflow end. The frame can include a plurality of pairs of axially extending first and second actuator posts. Each pair of the first and second actuator posts can be axially movable relative to one another. The frame can also include at least one rod extending through an inner bore of a selected second actuator post and coupled to a selected first actuator post paired with the selected second actuator post. Rotation of the rod can be configured to radially expand the frame from a radially compressed state to a radially expanded state. The frame can further include one or more axially extending support posts. At least two first actuator posts can include respective first movable markers and two second actuator posts paired with the at least two first actuator posts can include respective second movable markers to define pairs of first and second movable markers. The first and second movable markers of each pair of movable markers are spaced from each other by an axial distance that decreases as the frame is radially expanded. Positions of the first and second movable markers can be configured so that a difference between an axial distance of one of the pairs of movable markers and an axial distance of another one of the pairs of movable markers as the frame is radially expanded can indicate a degree of evenness of the frame's radial expansion. At least one support post can include a first fixed marker and a second fixed marker that are axially spaced apart from one another by a fixed distance so that an axial distance between each pair of the first and second movable markers can be compared to the fixed distance between the first fixed marker and the second fixed marker as the frame is radially expanded.

Certain aspects of the disclosure also concern an assembly including a prosthetic device having a frame and a delivery apparatus configured to deliver the prosthetic device into a target location. The frame can include a plurality of axially extending expansion and locking mechanisms and one or more axially extending support posts. Each expansion and locking mechanism can include a pair of first and second actuator posts that are axially movable relative to one another, and a rod extending through an inner bore of the second actuator post and coupled to the first actuator post. Rotation of the rod can be configured to radially expand the prosthetic device from a radially compressed state to a radially expanded state. At least two expansion and locking mechanisms can include respective pairs of first movable markers and second movable markers. The first movable markers can be respectively located on the first actuator posts of the at least two expansion and locking mechanisms and the second movable markers can be respectively located on the second actuator posts of the at least two expansion and locking mechanisms. Positions of the first and second movable markers can be configured so that a difference in axial distances between respective pairs of the first and second movable markers can indicate a degree of evenness of radial expansion of the frame. The delivery apparatus can include an outer shaft. A distal end portion of the outer shaft can be configured to house the prosthetic device in the radially compressed state. The delivery apparatus can also include at least one actuator assembly extending through the outer shaft and operatively connected to the frame and a handle connected to a proximal end of the outer shaft. The handle can include a first control mechanism configured to be actuated to move the prosthetic device out of a distal end of the outer shaft.

Certain aspects of the disclosure concern a method including radially expanding a prosthetic device from a radially compressed state to a radially expanded state and determining a diameter of the prosthetic device as the prosthetic device is radially expanded. The frame can include a pair of first and second fixed markers that are axially spaced apart from one another by a fixed distance and a pair of first and second movable markers that are axially movable relative to one another as the frame radially expands. Determining the diameter of the prosthetic device can include determining a distance between the first and second movable markers based on the fixed distance between the first and second fixed markers.

Certain aspects of the disclosure also concern a method including radially expanding a prosthetic device from a radially compressed state to a radially expanded state and monitoring an expansion evenness as the prosthetic device is radially expanded. The prosthetic device can include a frame which can include two or more pairs of first movable markers and second movable markers. Each pair of the first and second movable markers can be axially movable relative to one another as the frame radially expands. Monitoring the expansion evenness can include detecting a difference in axial distances between respective pairs of the first and second movable markers. A reduced difference can indicate an increase in expansion evenness and an increased distance can indicate a decrease in expansion evenness.

The above method(s) can be performed on a living animal or on a simulation, such as on a cadaver, cadaver heart, anthropomorphic ghost, simulator (e.g., with body parts, heart, tissue, etc. being simulated). The above method(s) are not a part of the invention.

In some examples, a prosthetic device/valve comprises one or more of the components recited in Examples 1-50 described in the section "Additional Examples of the Disclosed Technology" below.

The foregoing and other objects, features, and advantages of the disclosed technology will become more apparent from the following detailed description, which proceeds with reference to the accompanying figures.

### BRIEF DESCRIPTION OF THE DRAWINGS

FIG. 1A is a perspective view of one example of a prosthetic valve including a frame and a plurality of leaflets attached to the frame.
FIG. 1B is a perspective view of the prosthetic valve of FIG. 1A with an outer skirt disposed around the frame.
FIG. 2A is a perspective view of a frame for the prosthetic valve of FIG. 1A.
FIG. 2B is a front portion of the frame shown in FIG. 2A.
FIG. 3 is a side elevation view of a delivery apparatus for a prosthetic device, such as a prosthetic valve, according to one example.
FIG. 4 is a perspective view of a portion of an actuator of the prosthetic device of FIGS. 1-2 and an actuator assembly of a delivery apparatus, according to one example.
FIG. 5 is a perspective view of the actuator and actuator assembly of FIG. 4 with the actuator assembly physically coupled to the actuator.
FIG. 6 is a perspective view of a portion of a frame of a prosthetic device, according to another example.
FIG. 7 is a flattened view of a part of the frame of FIG. 6.

### DETAILED DESCRIPTION

### General Considerations

For purposes of this description, certain aspects, advantages, and novel features of examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language. Further, as used herein, "and/or" means "and" or "or," as well as "and" and "or."

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated examples. Such terms are not, however, intended to imply absolute relationships, positions, and/or orientations. For example, with respect to an object, an "upper" part can become a "lower" part simply by turning the object over. Nevertheless, it is still the same part and the object remains the same.

### Overview of the Disclosed Technology

Prosthetic valves disclosed herein can be radially compressible and expandable between a radially compressed state and a radially expanded state. Thus, the prosthetic valves can be crimped on or retained by an implant delivery apparatus in the radially compressed state while being advanced through a patient's vasculature on the delivery apparatus. The prosthetic valve can be expanded to the radially expanded state once the prosthetic valve reaches the implantation site. It is understood that the prosthetic valves disclosed herein may be used with a variety of implant delivery apparatuses and can be implanted via various delivery procedures, examples of which will be discussed in more detail later.

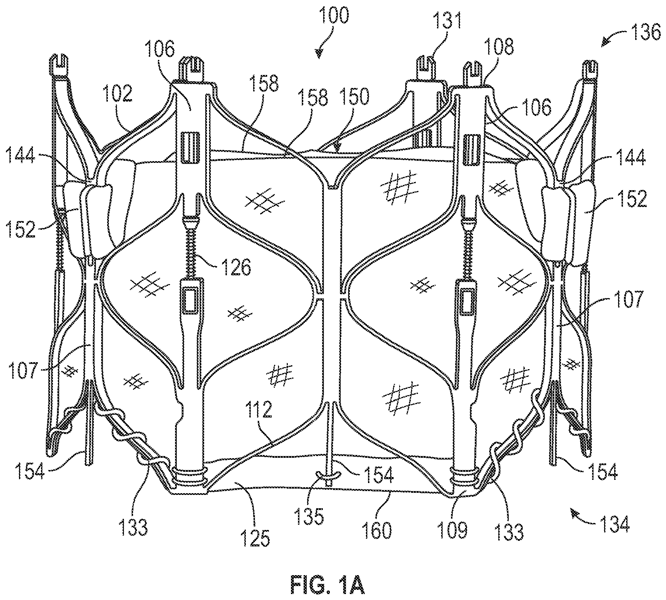

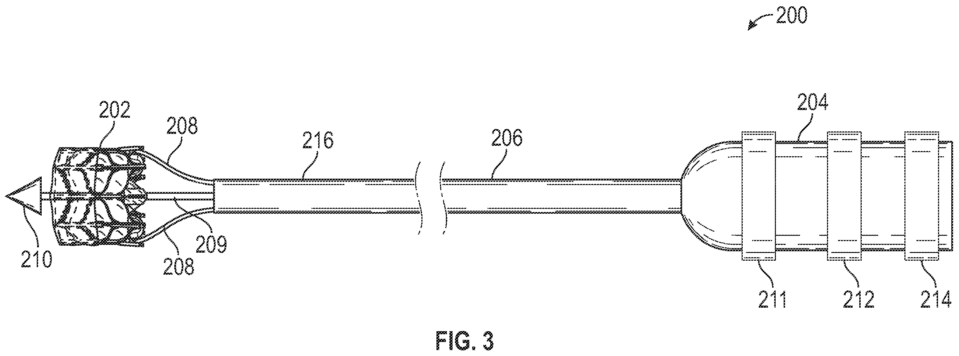

FIGS. 1A-2B illustrate an exemplary prosthetic device (e.g., prosthetic heart valve) that can be advanced through a patient's vasculature, such as to a native heart valve, by a delivery apparatus, such as the exemplary delivery apparatus shown in FIG. 3. The frame of the prosthetic heart valve can include one or more mechanical expansion and locking mechanisms that can be integrated into the frame, e.g., into axially extending posts of the frame. The mechanical expansion and/or locking mechanisms can be removably coupled to, and/or actuated by, the delivery apparatus to radially expand the prosthetic heart valve and lock the prosthetic heart valve in one or more radially expanded states. As described below, radial expansion of the prosthetic device can be monitored in real-time.

### Examples of Mechanically Expandable Prosthetic Valve

FIGS. 1A-2B show an exemplary prosthetic valve 100, according to one example. Any of the prosthetic valves disclosed herein are adapted to be implanted in the native aortic annulus, although in other examples they can be adapted to be implanted in other native annuluses of the heart (the pulmonary, mitral, and tricuspid valves). The disclosed prosthetic valves also can be implanted within vessels communicating with the heart, including a pulmonary artery (for replacing the function of a diseased pulmonary valve), or the superior vena cava or the inferior vena cava (for replacing the function of a diseased tricuspid valve), or various other veins, arteries and vessels of a patient. The disclosed prosthetic valves also can be implanted within a previously implanted prosthetic valve (which can be a prosthetic surgical valve or a prosthetic transcatheter heart valve) in a valve-in-valve procedure.

In some examples, the disclosed prosthetic valves can be implanted within a docking or anchoring device that is implanted within a native heart valve or a vessel. For example, in one example, the disclosed prosthetic valves can be implanted within a docking device implanted within the pulmonary artery for replacing the function of a diseased pulmonary valve, such as disclosed in U.S. Publication No. 2017/0231756. In another example, the disclosed prosthetic valves can be implanted within a docking device implanted within or at the native mitral valve, such as disclosed in PCT Publication No. WO2020/247907. In another example, the disclosed prosthetic valves can be implanted within a docking device implanted within the superior or inferior vena cava for replacing the function of a diseased tricuspid valve, such as disclosed in U.S. Publication No. 2019/0000615.

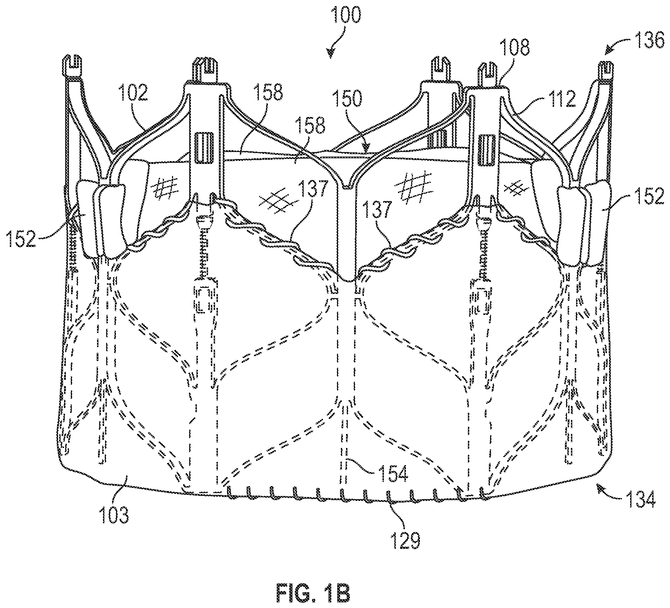

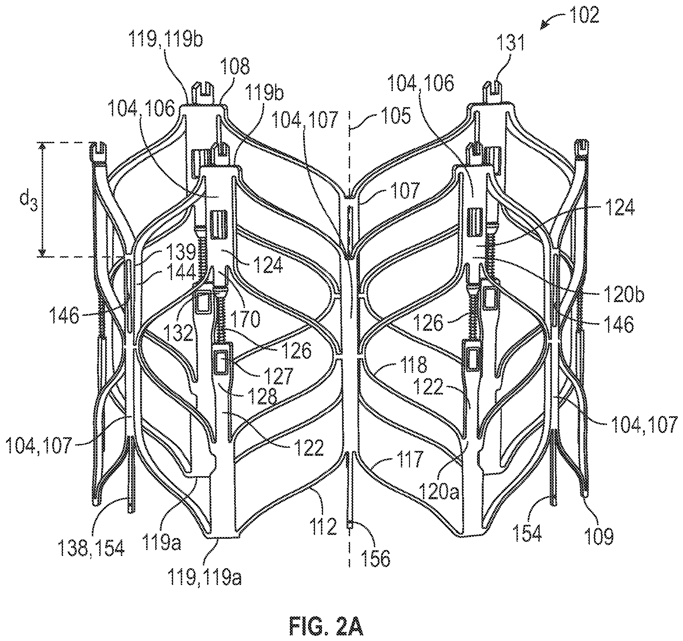

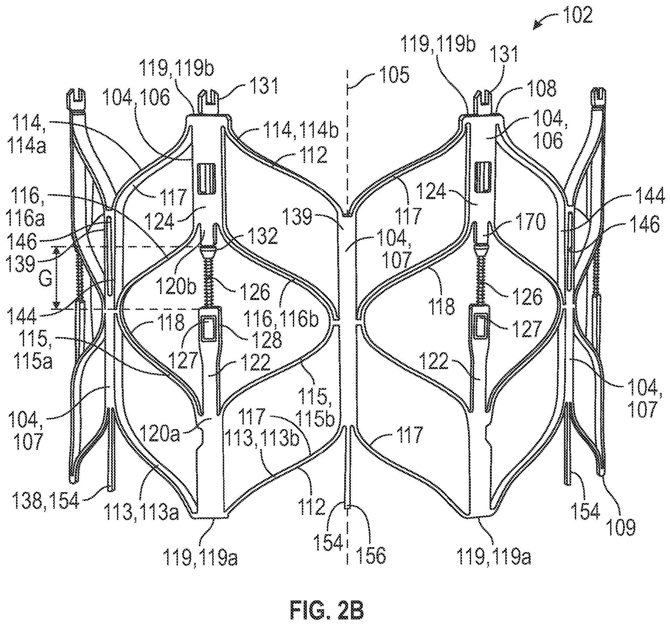

FIGS. 1A-2B illustrate an exemplary embodiment of a prosthetic device, such as a prosthetic valve 100 (which also may be referred to herein as "prosthetic heart valve 100") having a frame 102. FIGS. 2A-2B show the frame 102 by itself, while FIGS. 1A-1B show the frame 102 with a valvular structure 150 (which can comprise leaflets 158, as described further below) mounted within and to the annular frame 102. FIG. 1B additionally shows an optional skirt assembly comprising an outer skirt 103. While only one side of the frame 102 is depicted in FIG. 2B, it should be appreciated that the frame 102 forms an annular structure having an opposite side that is substantially identical to the portion shown in FIG. 1B, as shown in FIGS. 1A-2A.

As shown in FIGS. 1A and 1B, the valvular structure 150 is coupled to and supported inside the frame 102. The valvular structure 150 is configured to regulate the flow of blood through the prosthetic valve 100, from an inflow end portion 134 to an outflow end portion 136. The valvular structure 150 can include, for example, a leaflet assembly comprising one or more leaflets 158 made of flexible material. The leaflets 158 can be made from in whole or part, biological material, bio-compatible synthetic materials, or other such materials. Suitable biological material can include, for example, bovine pericardium (or pericardium from other sources). The leaflets 158 can be secured to one another at their adjacent sides to form commissures 152, each of which can be secured to a respective commissure support structure 144 (also referred to herein as "commissure supports") and/or to other portions of the frame 102, as described in greater detail below.

In the embodiment depicted in FIGS. 1A and 1B, the valvular structure 150 includes three leaflets 158, which can be arranged to collapse in a tricuspid arrangement. Each leaflet 158 can have an inflow edge portion 160 (which can also be referred to as a cusp edge portion) (FIG. 1A). The inflow edge portions 160 of the leaflets 158 can define an undulating, curved scallop edge that generally follows or tracks portions of struts 112 of frame 102 in a circumferential direction when the frame 102 is in the radially expanded configuration. The inflow edge portions 160 of the leaflets 158 can be referred to as a "scallop line."

The prosthetic valve 100 may include one or more skirts mounted around the frame 102. For example, as shown in FIG. 1B, the prosthetic valve 100 may include an outer skirt 103 mounted around an outer surface of the frame 102. The outer skirt 103 can function as a sealing member for the prosthetic valve 100 by sealing against the tissue of the native valve annulus and helping to reduce paravalvular leakage past the prosthetic valve 100. In some cases, an inner skirt (not shown) may be mounted around an inner surface of the frame 102. The inner skirt can function as a sealing member to prevent or decrease perivalvular leakage, to anchor the leaflets 158 to the frame 102, and/or to protect the leaflets 158 against damage caused by contact with the frame 102 during crimping and during working cycles of the prosthetic valve 100. In some examples, the inflow edge portions 160 of the leaflets 158 can be sutured to the inner skirt generally along the scallop line. The inner skirt can in turn be sutured to adjacent struts 112 of the frame 102. In other examples, as shown in FIG. 1A, the leaflets 158 can be sutured directly to the frame 102 or to a reinforcing member 125 (also referred to as a reinforcing skirt or connecting skirt) in the form of a strip of material (e.g., a fabric strip) which is then sutured to the frame 102, along the scallop line via stitches (e.g., whip stitches) 133.

The inner and outer skirts and the connecting skirt 125 can be formed from any of various suitable biocompatible materials, including any of various synthetic materials, including fabrics (e.g., polyethylene terephthalate fabric) or natural tissue (e.g., pericardial tissue). Further details regarding the use of skirts or sealing members in prosthetic valve can be found, for example, in U.S. Patent Publication No. 2020/0352711.

Further details regarding the assembly of the leaflet assembly and the assembly of the leaflets and the skirts to the frame can be found, for example, in U.S. Provisional Application Nos. 63/209,904, filed June 11, 2021, and 63/224,534, filed July 22, 2021. Further details of the construction and function of the frame 102 can be found in International Patent Application No. PCT/US2021/052745, filed September 30, 2021.

The frame 102, which is shown alone and in greater detail in FIGS. 2A and 2B, comprises and inflow end 109, an outflow end 108, and a plurality of axially extending posts 104. The axial direction of the frame 102 is indicated by a longitudinal axis 105, which extends from the inflow end 109 to the outflow end 108 (FIGS. 2A and 2B). Some of the posts 104 can be arranged in pairs of axially aligned first and second struts or posts 122, 124. An actuator 126 (such as the illustrated threaded rod or bolt) can extend through one or more pairs of posts 122, 124 to form an integral expansion and locking mechanism 106 (also referred to as the "actuator mechanism") configured to radially expand and compress the frame 102, as further described below. One or more of posts 104 can be configured as support posts 107.

The actuator mechanisms 106 (which can be used to radially expand and/or radially compress the prosthetic valve 100) can be integrated into the frame 102 of the prosthetic valve 100, thereby reducing the crimp profile and/or bulk of the prosthetic valve 100. Integrating the actuator mechanisms 106 (which can also be referred to herein as "expansion and locking mechanisms") into the frame 102 can also simplify the design of the prosthetic valve 100, making the prosthetic valve 100 less costly and/or easier to manufacture. In the illustrated example, an actuator 126 extends through each pair of axially aligned posts 122, 124. In other examples, one or more of the pairs of posts 122, 124 can be without a corresponding actuator.

The posts 104 can be coupled together by a plurality of circumferentially extending link members or struts 112. Each strut 112 extends circumferentially between adjacent posts 104 to connect all of the axially extending posts 104. As one example, the prosthetic valve 100 can include equal numbers of support posts 107 and pairs of actuator posts 122, 124, and the pairs of posts 122, 124 and the support posts 107 can be arranged in an alternating order such that each strut 112 is positioned between one of the pairs of posts 122, 124 and one of the support posts 107 (i.e., each strut 112 can be coupled on one end to one of the posts 122, 124 and can be coupled on the other end to one of the support posts 107). However, the prosthetic valve 100 can include different numbers of support posts 107 and pairs of posts 122, 124 and/or the pairs of posts 122, 124 and the support posts 107 can be arranged in a non-alternating order, in other examples.

As illustrated in FIG. 2B, the struts 112 can include a first row of struts 113 at or near the inflow end 109 of the prosthetic valve 100, a second row of struts 114 at or near the outflow end 108 of the prosthetic valve 100, and third and fourth rows of struts 115, 116, respectively, positioned axially between the first and second rows of struts 113, 114. The struts 112 can form and/or define a plurality of cells (i.e., openings) in the frame 102. For example, the struts 113, 114, 115, and 116 can at least partially form and/or define a plurality of first cells 117 and a plurality of second cells 118 that extend circumferentially around the frame 102. Specifically, each first cell 117 can be formed by two struts 113a, 113b of the first row of struts 113, two struts 114a, 114b of the second row of struts 114, and two of the support posts 107. Each second cell 118 can be formed by two struts 115a, 115b of the third row of struts 115 and two struts 116a, 116b of the fourth row of struts 116. As illustrated in FIGS. 2A and 2B, each second cell 118 can be disposed within one of the first cells 117 (i.e., the struts 115a-116b forming the second cells 118 are disposed between the struts forming the first cells 117 (i.e., the struts 113a, 113b and the struts 114a, 114b), closer to an axial midline of the frame 102 than the struts 113a-114b).

As illustrated in FIGS. 2A and 2B, the struts 112 of frame 102 can comprise a curved shape. Each first cell 117 can have an axially-extending hexagonal shape including first and second apices 119 (e.g., an inflow apex 119a and an outflow apex 119b). In examples where the delivery apparatus is releasably connected to the outflow apices 119b (as described below), each inflow apex 119a can be referred to as a "distal apex" and each outflow apex 119b can be referred to as a "proximal apex". Each second cell 118 can have a diamond shape including first and second apices 120 (e.g., distal apex 120a and proximal apex 120b). In some examples, the frame 102 comprises six first cells 117 extending circumferentially in a row, six second cells 118 extending circumferentially in a row within the six first cells 117, and twelve posts 104. However, in other embodiments, the frame 102 can comprise a greater or fewer number of first cells 117 and a correspondingly greater or fewer number of second cells 118 and posts 104.

As noted above, some of the posts 104 can be arranged in pairs of first post 122 and second post 124. The posts 122, 124 are aligned with each other along the length of the frame 102 and are axially separated from one another by a gap G (FIG. 2B) (those with actuators 126 can be referred to as actuator posts or actuator struts). Each first post 122 (i.e., the lower post shown in FIGS. 2A and 2B) can extend axially from the inflow end 109 of the prosthetic valve 100 toward the second post 124, and the second post 124 (i.e., the upper post shown in FIGS. 2A and 2B) can extend axially from the outflow end 108 of the prosthetic valve 100 toward the first post 122. For example, each first post 122 can be connected to and extend from an inflow apex 119a and each second post 124 can be connected to and extend from an outflow apex 119b. Each first post 122 and the second post 124 can include an inner bore configured to receive a portion of an actuator member, such as in the form of a substantially straight threaded rod 126 (or bolt) as shown in the illustrated example. The threaded rod 126 also may be referred to herein as actuator 126, actuator member 126, and/or screw actuator 126. In examples where the delivery apparatus can be releasably connected to the outflow end 108 of the frame 102, the first posts 122 can be referred to as distal posts or distal axial struts and the second posts 124 can be referred to as proximal posts or proximal axial struts.

Each threaded rod 126 extends axially through a corresponding first post 122 and second post 124. Each threaded rod 126 also extends through a bore of a nut 127 captured within a slot or window formed in an end portion 128 of the first post 122. The threaded rod 126 has external threads that engage internal threads of the bore of the nut 127. The inner bore of the second post 124 (through which the threaded rod 126 extends) can have a smooth and/or non-threaded inner surface to allow the threaded rod 126 to slide freely within the bore. Rotation of the threaded rod 126 relative to the nut 127 produces radial expansion and compression of the frame 102, as further described below.

In some examples, the threaded rod 126 can extend past the nut 127 toward the inflow end 109 of the frame 102 into the inner bore of the first post 122. The nut 127 can be held in a fixed position relative to the first post 122 such that the nut 127 does not rotate relative to the first post 122. In this way, whenever the threaded rod 126 is rotated (e.g., by a physician) the threaded rod 126 can rotate relative to both the nut 127 and the first post 122. The engagement of the external threads of the threaded rod 126 and the internal threads of the nut 127 prevent the rod 126 from moving axially relative to the nut 127 and the first post 122 unless the threaded rod 126 is rotated relative to the nut 127. Thus, the threaded rod 126 can be retained or held by the nut 127 and can only be moved relative to the nut 127 and/or the first post 122 by rotating the threaded rod 126 relative to the nut 127 and/or the first post 122. In other examples, in lieu of using the nut 127, at least a portion of the inner bore of the first post 122 can be threaded. For example, the bore along the end portion 128 of the first post 122 can comprise inner threads that engage the external threaded rod 126 such that rotation of the threaded rod causes the threaded rod 126 to move axially relative to the first post 122.

When a threaded rod 126 extends through and/or is otherwise coupled to a pair of axially aligned posts 122, 124, the pair of axially aligned posts 122, 124 and the threaded rod 126 can serve as one of the expansion and locking mechanisms 106. In some examples, a threaded rod 126 can extend through each pair of axially aligned posts 122, 124 so that all of the posts 122, 124 (with their corresponding rods 126) serve as expansion and locking mechanisms 106. As just one example, the prosthetic valve 100 can include six pairs of posts 122, 124, and each of the six pairs of posts 122, 124 with their corresponding rods 126 can be configured as one of the expansion and locking mechanisms 106 for a total of six expansion and locking mechanisms 106. In other examples, not all pairs of posts 122, 124 need be expansion and locking mechanisms (i.e., actuators). If a pair of posts 122, 124 is not used as an expansion and locking mechanism, a threaded rod 126 need not extend through the posts 122, 124 of that pair.

The threaded rod 126 can be rotated relative to the nut 127, the first post 122, and the second post 124 to axially foreshorten and/or axially elongate the frame 102, thereby radially expanding and/or radially compressing, respectively, the frame 102 (and therefore the prosthetic valve 100). Specifically, when the threaded rod 126 is rotated relative to the nut 127, the first post 122, and the second post 124, the first post 122 and the second post 124 can move axially relative to one another, thereby widening or narrowing the gap G (FIG. 2B) separating the posts 122, 124, and thereby radially compressing or radially expanding the prosthetic valve 100, respectively. Thus, the gap G (FIG. 2B) between the first post 122 and the second posts 124 narrows as the frame 102 is radially expanded and widens as the frame 102 is radially compressed.

The threaded rod 126 can extend proximally past the proximal end of the second post 124 and can include a head portion 131 at its proximal end that can serve at least two functions. First, the head portion 131 can removably or releasably couple the threaded rod 126 to a respective actuator assembly of a delivery apparatus that can be used to radially expand and/or radially compress the prosthetic valve 100 (e.g., the delivery apparatus 200 of FIG. 3, as described below). Second, the head portion 131 can prevent the second post 124 from moving proximally relative to the threaded rod 126 and can apply a distally directed force to the second post 124, such as when radially expanding the prosthetic valve 100. Specifically, the head portion 131 can have a width greater than a diameter of the inner bore of the second post 124 such that the head portion 131 is prevented from moving into the inner bore of the second post 124. Thus, as the threaded rod 126 is threaded farther into the nut 127, the head portion 131 of the threaded rod 126 draws closer to the nut 127 and the first post 122, thereby drawing the second post 124 towards the first post 122, and thereby axially foreshortening and radially expanding the prosthetic valve 100.

The threaded rod 126 also can include a stopper 132 (e.g., in the form of a nut, washer or flange) disposed thereon. The stopper 132 can be disposed on the threaded rod 126 such that it sits within the gap G. Further, the stopper 132 can be integrally formed on or fixedly coupled to the threaded rod 126 such that it does not move relative to the threaded rod 126. Thus, the stopper 132 can remain in a fixed axial position on the threaded rod 126 such that it moves in lockstep with the threaded rod 126.

Rotation of the threaded rod 126 in a first direction (e.g., clockwise) can cause corresponding axial movement of the first post 122 and the second post 124 toward one another, thereby decreasing the gap G and radially expanding the frame 102, while rotation of the threaded rod 126 in an opposite second direction causes corresponding axial movement of the first posts 122 and the second post 124 away from one another, thereby increasing the gap G and radially compressing the frame. When the threaded rod 126 is rotated in the first direction, the head portion 131 of the rod 126 bears against an adjacent surface of the frame (e.g., an outflow apex 119b), while the nut 127 and the first post 122 travel proximally along the threaded rod 126 toward the second post 124, thereby radially expanding the frame. As the frame 102 moves from a compressed configuration to an expanded configuration, the gap G between the first post 122 and the second post 124 can narrow.

When the threaded rod 126 is rotated in the second direction, the threaded rod 126 and the stopper 132 move toward the outflow end 108 of the frame until the stopper 132 abuts the inflow end 170 of the second post 124 (as shown in FIGS. 2A and 2B). Upon further rotation of the rod 126 in the second direction, the stopper 132 can apply a proximally directed force to the second post 124 to radially compress the frame 102. Specifically, during crimping/radial compression of the prosthetic valve 100, the threaded rod 126 can be rotated in the second direction (e.g., counterclockwise) causing the stopper 132 to push against (i.e., provide a proximally directed force to) the inflow end 170 of the second post 124, thereby causing the second post 124 to move away from the first post 122, and thereby axially elongating and radially compressing the prosthetic valve 100.

Thus, each of the second posts 124 can slide axially relative to a corresponding one of the first posts 122 but can be axially retained and/or restrained between the head portion 131 of a threaded rod 126 and a stopper 132. That is, each second post 124 can be restrained at its proximal end by the head portion 131 of the threaded rod 126 and at its distal end by the stopper 132. In this way, the head portion 131 can apply a distally directed force to the second post 124 to radially expand the prosthetic valve 100 while the stopper 132 can apply a proximally directed force to the second post 124 to radially compress the prosthetic valve 100. As explained above, radially expanding the prosthetic valve 100 axially foreshortens the prosthetic valve 100, causing an inflow end portion 134 and outflow end portion 136 of the prosthetic valve 100 (FIGS. 1A and 1B) to move towards one another axially, while radially compressing the prosthetic valve 100 axially elongates the prosthetic valve 100, causing the inflow and outflow end portions 134, 136 to move away from one another axially.

In other examples, the threaded rod 126 can be fixed against axial movement relative to the second post 124 (and the stopper 132 can be omitted) such that rotation of the threaded rod 126 in the first direction produces proximal movement of the nut 127 and radial expansion of the frame 102 and rotation of the threaded rod 126 in the second direction produces distal movement of the nut 127 and radial compression of the frame 102.

As also introduced above, some of the posts 104 can be configured as support posts 107. As shown in FIGS. 2A and 2B, the support posts 107 can extend axially between the inflow ends 109 and outflow ends 108 of the frame 102 and each can have an inflow end portion 138 and an outflow end portion 139. The outflow end portion 139 of one or more support posts 107 can include a commissure support structure or member 144. The commissure support structure 144 can comprise strut portions defining a commissure opening 146 therein.

The commissure opening 146 (which can also be referred to herein as a "commissure window 146") can extend radially through a thickness of the support post 107 and can be configured to accept a portion of a valvular structure 150 (e.g., a commissure 152) to couple the valvular structure 150 to the frame 102. For example, each commissure 152 can be mounted to a respective commissure support structure 144, such as by inserting a pair of commissure tabs of adjacent leaflets 158 through the commissure opening 146 and suturing the commissure tabs to each other and/or the commissure support structure 144. In some embodiments, the commissure opening 146 can be fully enclosed by the support post 107 such that a portion of the valvular structure 150 can be slid radially through the commissure opening 146, from an interior to an exterior of the frame 102, during assembly. In the illustrated embodiment, the commissure opening 146 has a substantially rectangular shape that is shaped and sized to receive commissure tabs of two adjacent leaflets therethrough. However, in other embodiments, the commissure opening can have any of various shapes (e.g., square, oval, square-oval, triangular, L-shaped, T-shaped, C-shaped, etc.).

The commissure openings 146 are spaced apart about the circumference of frame 102 (or angularly spaced apart about frame 102). The spacing may or may not be even. In one example, the commissure openings 146 are axially offset from the outflow end 108 of the frame 102 by an offset distance d3 (indicated in FIG. 2A). As an example, the offset distance d3 may be in a range from 2 mm to 6 mm. In general, the offset distance d3 should be selected such that when the leaflets are attached to the frame 102 via the commissure openings 146, the free edge portions (e.g., outflow edge portions) of the leaflets 158 will not protrude from or past the outflow end 108 of the frame 102.

The frame 102 can comprise any number of support posts 107, any number of which can be configured as commissure support structures 144. For example, the frame 102 can comprise six support posts 107, three of which are configured as commissure support structures 144. However, in other embodiments, the frame 102 can comprise more or less than six support posts 107 and/or more or less than three commissure support structures 144.

The inflow end portion 138 of each support post 107 can comprise an extension 154 (show as a cantilevered strut in FIGS. 2A and 2B) that extends toward the inflow end 109 of the frame 102. Each extension 154 can comprise an aperture 156 extending radially through a thickness of the extension 154. In some examples, the extension 154 can extend such that an inflow edge of the extension 154 aligns with or substantially aligns with the inflow end 109 of the frame 102. In use, the extension 154 can prevent or mitigate portions of an outer skirt from extending radially inwardly and thereby prevent or mitigate any obstruction of flow through the frame 102 caused by the outer skirt. The extensions 154 can further serve as supports to which portions of the inner and/or outer skirts and/or the leaflets and/or the connecting skirt 125 can be coupled. For example, sutures used to connect the inner and/or outer skirts and/or the leaflets and/or the connecting skirt 125 can be wrapped around the extensions 154 and/or can extend through apertures 156.

As an example, each extension 154 can have an aperture 156 (FIG. 2A) or other features to receive a suture or other attachment material for connecting an adjacent inflow edge portion 160 of a leaflet 158 (FIG. 1A), the outer skirt 103 (in FIG. 1B), the connecting skirt 125, and/or an inner skirt. In some examples, the inflow edge portion 160 of each leaflet 158 can be connected to a corresponding extension via a suture 135 (FIG. 1A).

In some examples, the outer skirt 103 can be mounted around the outer surface of frame 102 as shown in FIG. 1B and the inflow edge of the outer skirt 103 (lower edge in FIG. 1B) can be attached to the connecting skirt 125 and/or the inflow edge portions 160 of the leaflets 158 that have already been secured to frame 102 as well as to the extensions 154 of the frame by sutures 129. The outflow edge of the outer skirt 103 (the upper edge in FIG. 1B) can be attached to selected struts with stitches 137. In implementations where the prosthetic valve includes an inner skirt, the inflow edge of the inner skirt can be secured to the inflow edge portions 160 before securing the cusp edge portions to the frame so that the inner skirt will be between the leaflets and the inner surface of the frame. After the inner skirt and leaflets are secured in place, then the outer skirt can be mounted around the frame as described above.

The frame 102 can be a unitary and/or fastener-free frame that can be constructed from a single piece of material (e.g., Nitinol, stainless steel or a cobalt-chromium alloy), such as in the form of a tube. The plurality of cells can be formed by removing portions (e.g., via laser cutting) of the single piece of material. The threaded rods 126 can be separately formed and then be inserted through the bores in the second (proximal) posts 124 and threaded into the threaded nuts 127.

In some examples, the frame 102 can be formed from a plastically-expandable material, such as stainless steel or a cobalt-chromium alloy. When the frame is formed from a plastically-expandable material, the prosthetic valve 100 can be placed in a radially compressed state along the distal end portion of a delivery apparatus for insertion into a patient's body. When at the desired implantation site, the frame 102 (and therefore the prosthetic valve 100) can be radially expanded from the radially compressed state to a radially expanded state via actuation of actuation assemblies of the delivery apparatus (as further described below), which rotate the rods 126 to produce expansion of the frame 102. During delivery to the implantation site, the prosthetic valve 100 can be placed inside of a delivery capsule (sheath) to protect against the prosthetic valve contacting the patient's vasculature, such as when the prosthetic valve is advanced through a femoral artery. The capsule can also retain the prosthetic valve in a compressed state having a slightly smaller diameter and crimp profile than may be otherwise possible without a capsule by preventing any recoil (expansion) of the frame once it is crimped onto the delivery apparatus.

In other examples, the frame 102 can be formed from a self-expandable material (e.g., Nitinol). When the frame 102 is formed from a self-expandable material, the prosthetic valve can be radially compressed and placed inside the capsule of the delivery apparatus to maintain the prosthetic valve in the radially compressed state while it is being delivered to the implantation site. When at the desired implantation site, the prosthetic valve is deployed or released from the capsule. In some examples, the frame (and therefore the prosthetic valve) can partially self-expand from the radially compressed state to a partially radially expanded state. The frame 102 (and therefore the prosthetic valve 100) can be further radially expanded from the partially expanded state to a further radially expanded state via actuation of actuation assemblies of the delivery apparatus (as further described below), which rotate the rods 126 to produce expansion of the frame.

### Examples of Delivery Apparatus

As introduced above, the threaded rods 126 can removably couple the prosthetic valve 100 to actuator assemblies of a delivery apparatus. Referring to FIG. 3, it illustrates an exemplary delivery apparatus 200 for delivering a prosthetic device or valve 202 (e.g., prosthetic valve 100) to a desired implantation location. The prosthetic valve 202 can be releasably coupled to the delivery apparatus 200. It should be understood that the delivery apparatus 200 and other delivery apparatuses disclosed herein can be used to implant prosthetic devices other than prosthetic valves, such as stents or grafts.

The delivery apparatus 200 in the illustrated embodiment generally includes a handle 204, a first elongated shaft 206 (which comprises an outer shaft in the illustrated example) extending distally from the handle 204, at least one actuator assembly 208 extending distally through the first shaft 206, a second elongated shaft 209 (which comprises an inner shaft in the illustrated example) extending through the first shaft 206, and a nosecone 210 coupled to a distal end portion of the second shaft 209. The second shaft 209 and the nosecone 210 can define a guidewire lumen for advancing the delivery apparatus through a patient's vasculature over a guidewire. The at least one actuator assembly 208 can be configured to radially expand and/or radially collapse the prosthetic valve 202 when actuated, such as by one or more knobs 211, 212, 214 included on the handle 204 of the delivery apparatus 200.

Though the illustrated embodiment shows two actuator assemblies 208 for purposes of illustration, it should be understood that one actuator assembly 208 can be provided for each actuator (e.g., actuator or threaded rod 126) on the prosthetic valve. For example, three actuator assemblies 208 can be provided for a prosthetic valve having three actuators. **In** other embodiments, a greater or fewer number of actuator assemblies can be present.

In some embodiments, a distal end portion 216 of the shaft 206 can be sized to house the prosthetic valve in its radially compressed, delivery state during delivery of the prosthetic valve through the patient's vasculature. In this manner, the distal end portion 216 functions (and also can be referred to) as a delivery sheath or capsule for the prosthetic valve during delivery,

The actuator assemblies 208 can be releasably coupled to the prosthetic valve 202. For example, in the illustrated embodiment, each actuator assembly 208 can be coupled to a respective actuator (e.g., threaded rod 126) of the prosthetic valve 202. Each actuator assembly 208 can comprise a support tube and an actuator member. When actuated, the actuator assembly can transmit pushing and/or pulling forces to portions of the prosthetic valve to radially expand and collapse the prosthetic valve as previously described. The actuator assemblies 208 can be at least partially disposed radially within, and extend axially through, one or more lumens of the first shaft 206. For example, the actuator assemblies 208 can extend through a central lumen of the shaft 206 or through separate respective lumens formed in the shaft 206.

The handle 204 of the delivery apparatus 200 can include one or more control mechanisms (e.g., knobs or other actuating mechanisms) for controlling different components of the delivery apparatus 200 in order to expand and/or deploy the prosthetic valve 202. For example, in the illustrated embodiment the handle 204 comprises first, second, and third knobs 211, 212, and 214, respectively.

The first knob 211 can be a rotatable knob configured to produce axial movement of the first shaft 206 relative to the prosthetic valve 202 in the distal and/or proximal directions in order to deploy the prosthetic valve from the delivery sheath 216 once the prosthetic valve has been advanced to a location at or adjacent the desired implantation location with the patient's body. For example, rotation of the first knob 211 in a first direction (e.g., clockwise) can retract the sheath 216 proximally relative to the prosthetic valve 202 and rotation of the first knob 211 in a second direction (e.g., counter-clockwise) can advance the sheath 216 distally. In other embodiments, the first knob 211 can be actuated by sliding or moving the first knob 211 axially, such as pulling and/or pushing the knob. In other embodiments, actuation of the first knob 211 (rotation or sliding movement of the first knob 211) can produce axial movement of the actuator assemblies 208 (and therefore the prosthetic valve 202) relative to the delivery sheath 216 to advance the prosthetic valve distally from the sheath 216.

The second knob 212 can be a rotatable knob configured to produce radial expansion and/or compression of the prosthetic valve 202. For example, rotation of the second knob 212 can rotate the threaded rods of the prosthetic valve 202 via the actuator assemblies 208. Rotation of the second knob 212 in a first direction (e.g., clockwise) can radially expand the prosthetic valve 202 and rotation of the second knob 212 in a second direction (e.g., counter-clockwise) can radially collapse the prosthetic valve 202. In other embodiments, the second knob 212 can be actuated by sliding or moving the second knob 212 axially, such as pulling and/or pushing the knob.

The third knob 214 can be a rotatable knob operatively connected to a proximal end portion of each actuator assembly 208. The third knob 214 can be configured to retract an outer sleeve or support tube of each actuator assembly 208 to disconnect the actuator assemblies 208 from the proximal portions of the actuators of the prosthetic valve (e.g., threaded rod). Once the actuator assemblies 208 are uncoupled from the prosthetic valve 202, the delivery apparatus 200 can be removed from the patient, leaving just the prosthetic valve 202 in the patient.

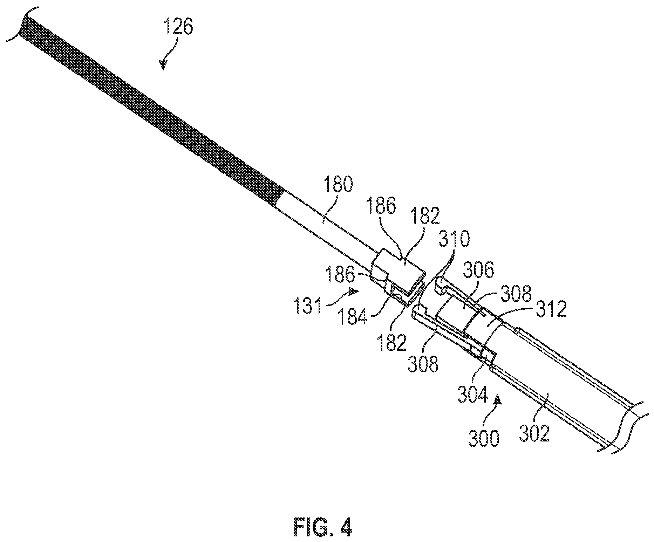

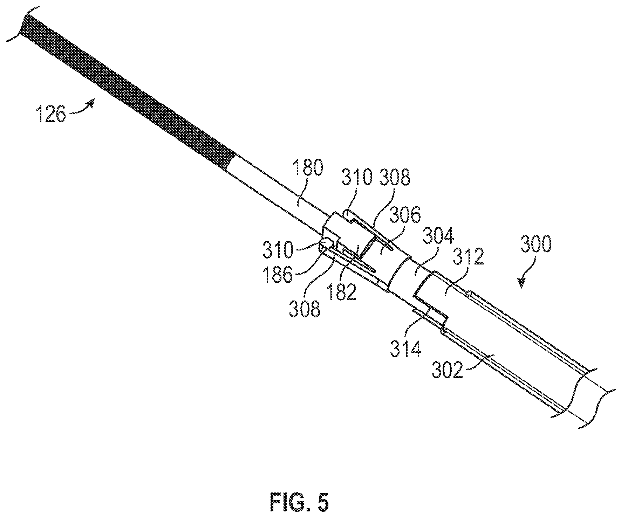

Referring to FIGS. 4-5, they illustrate how each of the threaded rods 126 of the prosthetic device 100 can be removably coupled to an exemplary actuator assembly 300 (e.g., actuator assemblies 208) of a delivery apparatus (e.g., delivery apparatus 200). Specifically, FIG. 5 illustrates how one of the threaded rods 126 can be coupled to an actuator assembly 300, while FIG. 4 illustrates how the threaded rod 126 can be detached from the actuator assembly 300.

As introduced above, an actuator assembly 300 can be coupled to the head portion 131 of each threaded rod 126. The head portion 131 can be included at a proximal end portion 180 of the threaded rod 126 and can extend proximally past a proximal end of the second post 124 (FIG. 2A). The head portion 131 can comprise first and second protrusions 182 defining a channel or slot 184 between them, and one or more shoulders 186. As discussed above, the head portion 131 can have a width greater than a diameter of the inner bore of the second post 124 such that the head portion 131 is prevented from moving into the inner bore of the second post 124 and such that the head portion 131 abuts the outflow end 108 of the frame 102. In particular, the head portion 131 can abut an outflow apex 119b of the frame 102. The head portion 131 can be used to apply a distally-directed force to the second post 124, for example, during radial expansion of the frame 102.

Each actuator assembly 300 can comprise a first actuation member configured as a support tube or outer sleeve 302 and a second actuation member configured as a driver 304. The driver 304 can extend through the outer sleeve 302. The outer sleeve 302 is shown transparently in FIGS. 4-5 for purposes of illustration. The distal end portions of the outer sleeve 302 and driver 304 can be configured to engage or abut the proximal end of the threaded rod 126 (e.g., the head portion 131) and/or the frame 102 (e.g., the apex 119b). The proximal portions of the outer sleeve 302 and driver 304 can be operatively coupled to the handle of a delivery apparatus (e.g., handle 204). The delivery apparatus in this embodiment can include the same features described previously for delivery apparatus 200. **In** particular embodiments, the proximal end portions of each driver 304 can be operatively connected to the knob 212 such that rotation of the knob 212 (clockwise or counterclockwise) causes corresponding rotation of the drivers 304. The proximal end portions of each outer sleeve 302 can be operatively connected to the knob 214 such that rotation of the knob 214 (clockwise or counterclockwise) causes corresponding axial movement of the sleeves 302 (proximally or distally) relative to the drivers 304. In other embodiments, the handle can include electric motors for actuating these components.

The distal end portion of the driver 304 can comprise a central protrusion 306 configured to extend into the slot 184 of the threaded rod 126, and one or more flexible elongated elements or arms 308 including protrusions or teeth 310 configured to be releasably coupled to the shoulders 186 of the threaded rod 126. The protrusions 310 can extend radially inwardly toward a longitudinal axis of the second actuation member 304. As shown in FIGS. 4-5, the elongated elements 308 can be configured to be biased radially outward to an expanded state, for example, by shape setting the elements 308.

As shown in FIG. 5, to couple the actuator assembly 300 to the threaded rod 126, the driver 304 can be positioned such that the central protrusion 306 is disposed within the slot 184 (FIG. 4) and such that the protrusions 310 of the elongated elements 308 are positioned distally to the shoulders 186. As the outer sleeve 302 is advanced (e.g., distally) over the driver 304, the sleeve 302 compresses the elongated elements 308 so that they abut and/or snap over the shoulders 186, thereby coupling the actuator assembly 300 to the threaded rod 126. Thus, the outer sleeve 302 effectively squeezes and locks the elongated elements 308 and the protrusions 310 of the driver 304 into engagement with (i.e., over) the shoulders 186 of the threaded rod 126, thereby coupling the driver 304 to the threaded rod 126.

Because the central protrusion 306 of the driver 304 extends into the slot 184 of the threaded rod 126 when the driver 304 and the threaded rod 126 are coupled, the driver 304 and the threaded rod 126 can be rotational locked such that they can co-rotate. So coupled, the driver 304 can be rotated (e.g., using knob 212 of the handle of the delivery apparatus 200) to cause corresponding rotation of the threaded rod 126 to radially expand or radially compress the prosthetic device. The central protrusion 306 can be configured (e.g., sized and shaped) such that it is advantageously spaced apart from the inner walls of the outer sleeve 302, such that the central protrusion 306 does not frictionally contact the outer sleeve 302 during rotation. Though in the illustrated embodiment the central protrusion 306 has a substantially rectangular shape in cross-section, in other embodiments, the protrusion 306 can have any of various shapes, for example, square, triangular, oval, etc. The slot 184 can be correspondingly shaped to receive the protrusion 306.

The outer sleeve 302 can be advanced distally relative to the driver 304 past the elongated elements 308, until the outer sleeve 302 engages the frame 102 (e.g., a second post 124 of the frame 102). The distal end portion of the outer sleeve 302 also can comprise first and second support extensions 312 defining gaps or notches 314 between the extensions 312. The support extensions 312 can be oriented such that, when the actuator assembly 300 is coupled to a respective threaded rod 126, the support extensions 312 extend partially over an adjacent end portion (e.g., the upper end portion) of one of the second posts 124 on opposite sides of the post 124. The engagement of the support extensions 312 with the frame 102 in this manner can counter-act rotational forces applied to the frame 102 by the rods 126 during expansion of the frame 102. In the absence of a counter-force acting against these rotational forces, the frame can tend to "jerk" or rock in the direction of rotation of the rods when they are actuated to expand the frame. The illustrated configuration is advantageous in that outer sleeves, when engaging the proximal posts 124 of the frame 102, can prevent or mitigate such jerking or rocking motion of the frame 102 when the frame 102 is radially expanded.

To decouple the actuator assembly 300 from the prosthetic device 100, the sleeve 302 can be withdrawn proximally relative to the driver 304 until the sleeve 302 no longer covers the elongated elements 308 of the driver 304. As described above, the sleeve 302 can be used to hold the elongated elements 308 against the shoulders 186 of the threaded rod 126 since the elongated elements 308 can be naturally biased to a radial outward position where the elongated elements 308 do not engage the shoulders 186 of the threaded rod 126. Thus, when the sleeve 302 is withdrawn such that it no longer covers/constrains the elongated elements 308, the elongated elements 308 can naturally and/or passively deflect away from, and thereby release from, the shoulders 186 of the threaded rod 126, thereby decoupling the driver 304 from the threaded rod 126.

The sleeve 302 can be advanced (moved distally) and/or retracted (moved proximally) relative to the driver 304 via a control mechanism (e.g., knob 214) on the handle 204 of the delivery apparatus 200, by an electric motor, and/or by another suitable actuation mechanism. For example, the physician can turn the knob 214 in a first direction to apply a distally directed force to the sleeve 302 and can turn the knob 214 in an opposite second direction to apply a proximally directed force to the sleeve 302. Thus, when the sleeve 302 does not abut the prosthetic device and the physician rotates the knob 214 in the first direction, the sleeve 302 can move distally relative to the driver 304, thereby advancing the sleeve 302 over the driver 304. When the sleeve 302 does abut the prosthetic device, the physician can rotate the knob 214 in the first direction to push the entire prosthetic device distally via the sleeve 302. Further, when the physician rotates the knob 214 in the second direction the sleeve 302 can move proximally relative to the driver 304, thereby withdrawing/retracting the sleeve 302 from the driver 304.

### Exemplary Delivery Techniques

For implanting a prosthetic valve within the native aortic valve via a transfemoral delivery approach, the prosthetic valve is mounted in a radially compressed state along the distal end portion of a delivery apparatus. The prosthetic valve and the distal end portion of the delivery apparatus are inserted into a femoral artery and are advanced into and through the descending aorta, around the aortic arch, and through the ascending aorta. The prosthetic valve is positioned within the native aortic valve and radially expanded (e.g., by inflating a balloon, actuating one or more actuators of the delivery apparatus, or deploying the prosthetic valve from a sheath to allow the prosthetic valve to self-expand). Alternatively, a prosthetic valve can be implanted within the native aortic valve in a transapical procedure, whereby the prosthetic valve (on the distal end portion of the delivery apparatus) is introduced into the left ventricle through a surgical opening in the chest and the apex of the heart and the prosthetic valve is positioned within the native aortic valve. Alternatively, in a transaortic procedure, a prosthetic valve (on the distal end portion of the delivery apparatus) are introduced into the aorta through a surgical incision in the ascending aorta, such as through a partial **J-**sternotomy or right parasternal mini-thoracotomy, and then advanced through the ascending aorta toward the native aortic valve.

For implanting a prosthetic valve within the native mitral valve via a transseptal delivery approach, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, into the right atrium, across the atrial septum (through a puncture made in the atrial septum), into the left atrium, and toward the native mitral valve. Alternatively, a prosthetic valve can be implanted within the native mitral valve in a transapical procedure, whereby the prosthetic valve (on the distal end portion of the delivery apparatus) is introduced into the left ventricle through a surgical opening in the chest and the apex of the heart and the prosthetic valve is positioned within the native mitral valve.

For implanting a prosthetic valve within the native tricuspid valve, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, and into the right atrium, and the prosthetic valve is positioned within the native tricuspid valve. A similar approach can be used for implanting the prosthetic valve within the native pulmonary valve or the pulmonary artery, except that the prosthetic valve is advanced through the native tricuspid valve into the right ventricle and toward the pulmonary valve/pulmonary artery.

Another delivery approach is a trans-atrial approach whereby a prosthetic valve (on the distal end portion of the delivery apparatus) is inserted through an incision in the chest and an incision made through an atrial wall (of the right or left atrium) for accessing any of the native heart valves. Atrial delivery can also be made intravascularly, such as from a pulmonary vein. Still another delivery approach is a trans-ventricular approach whereby a prosthetic valve (on the distal end portion of the delivery apparatus) is inserted through an incision in the chest and an incision made through the wall of the right ventricle (typically at or near the base of the heart) for implanting the prosthetic valve within the native tricuspid valve, the native pulmonary valve, or the pulmonary artery.

In all delivery approaches, the delivery apparatus can be advanced over a guidewire previously inserted into a patient's vasculature. Moreover, the disclosed delivery approaches are not intended to be limited. Any of the prosthetic valves disclosed herein can be implanted using any of various delivery procedures and delivery devices known in the art.

The treatment techniques, methods, steps, etc. described or suggested herein or in references incorporated herein can be performed on a living animal or on a non-living simulation, such as on a cadaver, cadaver heart, anthropomorphic ghost, simulator (e.g., with the body parts, tissue, etc. being simulated), etc.

### Another Example Frame for a Prosthetic Device

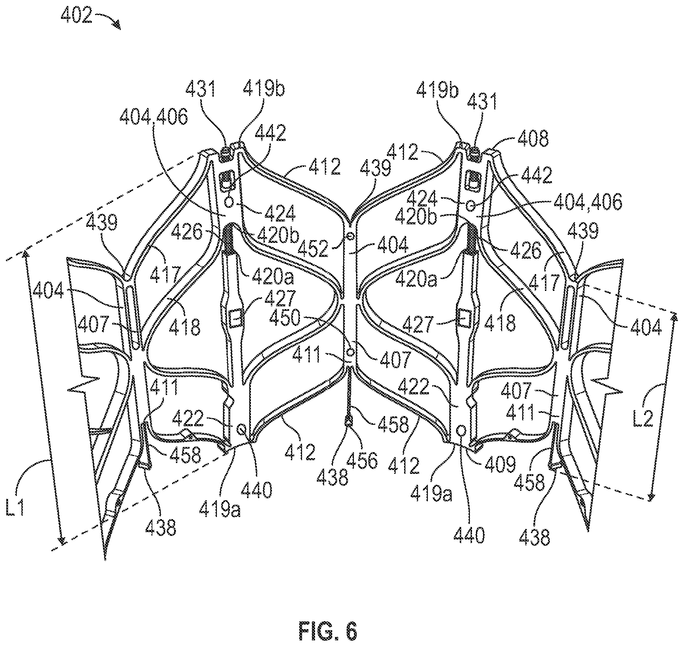

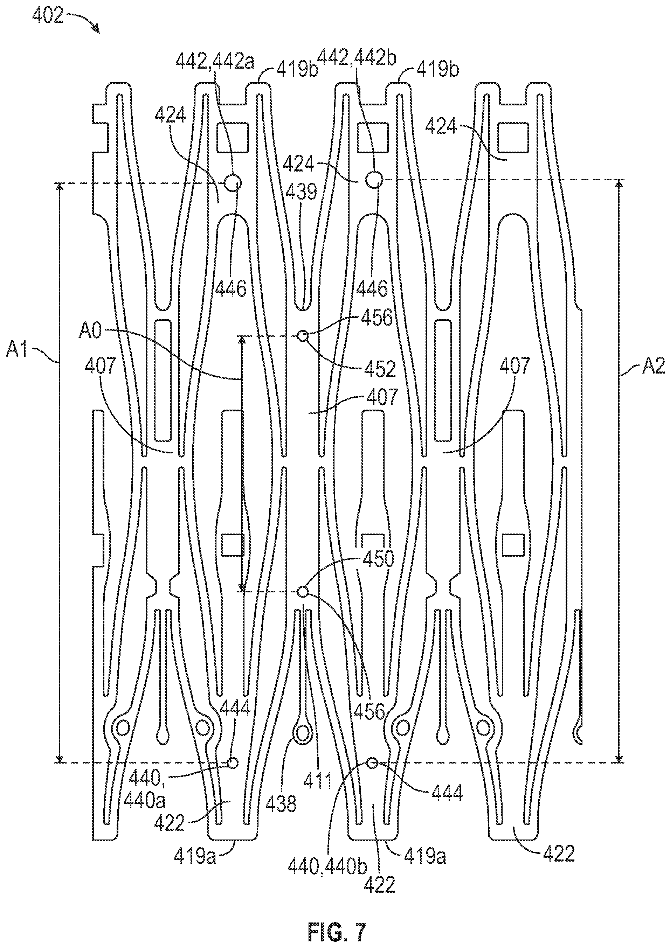

FIG. 6 shows perspective view of a portion of a frame 402, according to another example. A flattened part of the frame 402 is shown in FIG. 7. Similar to the frame 102, the frame 402 can be included in a prosthetic device, such as the prosthetic valve 100. In certain examples, a prosthetic heart valve can include the frame 402, a leaflet structure 150 (including a plurality of leaflets 158), and an outer skirt 103. The leaflets 158 and the outer skirt 103 can be mounted to the frame 402 in the same manner as described above for the prosthetic valve 100.

The frame 402 can have similar structure as the frame 102. For example, the frame 402 can have an inflow end 409, an outflow end 408, and a plurality of axially extending posts 404. Some of the posts 404 can be arranged in pairs of axially aligned first and second actuator posts 422, 424.

In any of the examples described herein, the first actuator posts 422 can have the same length, and the second actuator posts 424 can also have the same length. The axial length of the first actuator posts 422 can be the same as or different from the axial length of the second actuator posts 424.

A screw actuator or rod 426 (similar to the threaded rod 126) can extend through one or more pairs of actuator posts 422, 424 to form an integral expansion and locking mechanism or actuator mechanism 406 configured to radially expand and compress the frame 402, based on the same mechanisms (e.g., rotation of the rod 426) described above.

For example, a first actuator post 422 can house a nut 427 that is held in a fixed position relative to the first actuator post 422 such that the nut 427 does not rotate relative to the first actuator post 422. The threaded portion of the rod 426 can extend through and engage with internal threads of the nut 427 such that rotation of the rod 426 can cause the rod 426 to move axially relative to both the nut 427 and the first actuator post 422. For example, the rod 426 can be configured to be rotated in a first direction (e.g., clockwise or counter-clockwise) to thread farther into the threaded bore to axially compress and radially expand the frame 402, and the rod 426 can be configured to be rotated in an opposite second direction (e.g., counter-clockwise or clockwise) to axially elongate and radially compress the frame 402. In lieu of or in addition to the threaded but, the first actuator post 422 can have a threaded bore that engages the threaded portion of the rod 426.

In some examples, a rod 426 can extend through each pair of axially aligned actuator posts 422, 424 so that all of the actuator posts 422, 424 (with their corresponding rods 426) serve as expansion and locking mechanisms 406. For example, the frame 402 can have six pairs of actuator posts 422, 424, and each of the six pairs of actuator posts 422, 424 with their corresponding rods 426 can be configured as one of the expansion and locking mechanisms 406 for a total of six expansion and locking mechanisms 406. In other examples, not all pairs of actuator posts 422, 424 need be expansion and locking mechanisms (i.e., actuators).

As shown, each first actuator post 422 can have a first end 419a and a second end 420a, the second end 420a being closer to the corresponding (paired) actuator post 424 than the first end 419a. Each second actuator post 424 can have a first end 420b and a second end 419b, the first end 420b being closer to the corresponding (paired) actuator post 422 than the second end 419b. Thus, as one or more rods 426 rotate to radially expand or compress the frame 402, each pair of actuator posts 422, 424 can move axially relative to one another. As such, the axial distance L1 between the first end 419a of the actuator post 422 and the second end 419b of the paired actuator post 424 can change accordingly. For example, the distance L1 increases when the frame 402 is radially compressed, and decreases when the frame 402 is radially expanded.

In certain examples, the rod 426 can include a stopper (e.g., stopper 132; not shown in FIG. 6) positioned adjacent the first end 420b of the actuator post 424 and a head portion 431 positioned adjacent the second end 419b of the actuator post 424 to apply proximally and distally directed forces to the post 424 during radial compression and expansion, respectively, of the frame, as previously described for the frame 102.

Likewise, some of the posts 404 can be configured as support posts 407 which extend axially (e.g., parallel to the pairs of actuator posts 422, 424) around a circumference of the frame 402. The posts 404 can be coupled together by a plurality of circumferentially extending struts 412. Each strut 412 extends circumferentially between adjacent posts 404 to connect all of the axially extending posts 404. As one example, the frame 402 can include equal numbers of support posts 407 and pairs of actuator posts 422, 424, and the pairs of actuator posts 422, 424 and the support posts 407 can be arranged in an alternating order such that each strut 412 is positioned between one of the pairs of actuator posts 422, 424 and one of the support posts 407 (i.e., each strut 412 can be coupled on one end to one of the actuator posts 422, 424 and can be coupled on the other end to one of the support posts 407). However, the frame 402 can include different numbers of support posts 407 and pairs of actuator posts 422, 424 and/or the pairs of actuator posts 422, 424 and the support posts 407 can be arranged in a non-alternating order, in other examples. Similarly, the struts 412 can form and/or define a plurality of first cells 417 (which can be referred to as outer cells in some examples) and a plurality of second cells 418 (which can be referred to as inner cells in some examples) of the frame 402 such that each second cell 418 can be disposed within one of the first cells 417. Each pair of a first cell 417 and a second cell 418 can be referred to as a "cell column," which in the illustrated example, spans the height of the frame from the inflow end 409 to the outflow end 410.

Each of the support posts 407 can have first end 438 and a second end 439, the second end 439 being closer to the outflow end 408 than the first end 438. In the depicted example, each support post 407 can have an extension 458 which can extend such that the first end 438 can align with or substantially align with the inflow end 409 of the frame 402. In certain examples, at least some of the extensions 458 can have respective apertures 456 extending through a thickness of the extensions 458. In other examples, at least some of the support posts 407 may not have the extensions 458, e.g., the first end 438 may terminate at a junction 411 between two adjacent struts 412 and thus is axially offset from the inflow end 409. In the depicted example, each of the support post 407 has a fixed axial length L2 (in contrast to variable L1) measured between the first end 438 and the second end 439.

The delivery apparatus 200 described above can be used to deliver and deploy a prosthetic device comprising the frame 402. For example, one or more actuator assemblies 208 can be releasably coupled to respective actuator or rod 426 of the frame 402, and one or more control mechanisms (e.g., 211, 212, 214, etc.) on the handle 204 can control different components of the delivery apparatus 200 in order to expand and/or deploy the prosthetic device comprising the frame 402, as described above.

As described further below, the frame 402 can include one or more markers configured to allow an operator (e.g., a physician) to monitor the radial expansion process of the frame 402 (and the prosthetic device comprising the frame) in real-time during the operation.

### Exemplary Techniques for Monitoring Evenness of Frame's Radial Expansion

According to certain examples, the frame 402 can include two or more pairs of a first movable marker 440 and a second movable marker 442, wherein the pairs of movable markers are circumferentially spaced apart from each other. The first and second movable markers 440, 442 of each pair are axially aligned and axially movable relative to one another as the frame 402 radially expands.

For example, each first movable marker 440 can be located on a respective first actuator post 422, and each second movable marker 442 can be located on a respective second actuator post 424. Thus, as a pair of actuator posts 422, 424 move axially relative to one another, the first and second movable markers 440, 442 located on the pair of actuator posts 422, 424 also move axially relative to one another.

**In** some examples, each pair of actuator posts 422, 424 can comprise a corresponding pair of first and second movable markers 440, 442. **In** other examples, the first and second movable markers 440, 442 are located on some pairs of actuator posts 422, 424, but not on other pairs of actuator posts 422, 424. For example, if the frame 402 has six pairs of actuator posts 422, 424, the first and second movable markers 440, 442 can be located on any selected two, three, four, or five pairs, or on all six pairs of the actuator posts 422, 424.

As described herein, positions of the first and second movable markers 440, 442 can be configured so that a difference in axial distances between respective pairs of the first movable markers 440 and second movable markers 442 can indicate a degree of evenness of the frame's radial expansion.

For example, FIG. 7 shows two first movable markers 440a, 440b and the corresponding two second movable markers 442a, 442b, respectively located on two adjacent pairs of first and second actuator posts 422, 424.

In the depicted example, the first movable markers 440a, 440b are located on the respective first actuator posts 422 at a location that is adjacent to the respective first end 419a. The second movable markers 442a, 442b are located on the respective second actuator posts 424 at a location that is adjacent to the respective second end 419b. The distance between each of the first movable markers 440a, 440b to the respective first end 419a can be the same, and the distance between each of the second movable markers 442a, 442b to the respective second end 419b can also be the same.

As the frame 402 radially expands, the axial distance between the first and second movable markers 440a, 442a (denoted as A1) and the axial distance between the first and second movable markers 440b, 442b (denoted as A2) both decrease as the pair of first and second actuator posts 422, 424 move toward each other. Conversely, as the frame 402 radially compresses, both A1 and A2 increase as the pair of first and second actuator posts 422, 424 move away from each other.

Thus, a difference in axial differences A1 and A2 along different cell columns can indicate a degree of evenness of the frame's radial expansion. Specifically, a reduced difference between A1 and A2 can indicate an increase in expansion evenness (e.g., both cell columns are expanding at the same rate or substantially the same rate), whereas an increased difference between A1 and A2 can indicate a decrease in expansion evenness (e.g., the cell columns are expanding at different rates). For example, if A1 is the same or substantially the same as A2, it can indicate the frame 402 is expanded evenly. In contrast, if the difference between A1 and A2 exceeds a predefined threshold, it can indicate the frame 402 is expanded unevenly, which can be deemed undesirable. More specifically, if the difference between A1 and A2 exceeds the predefined threshold, this can indicate that the expansion of the frame along a first cell column (a pair of cells 417, 418) corresponding to A1 has exceeded the expansion of the frame along a second cell column corresponding to A2 (or vice versa) beyond what is acceptable to achieve full and complete deployment of the frame.

As noted above, in some examples, a pair of movable markers 440, 442 can be provided on each pair of actuator posts 422, 424. Thus, each cell column can be provided with a pair of movable markers 440, 422 to compare the rate of expansion and extent of expansion of each cell column to each other to determine if the frame is being deployed in an even manner.

Uneven radial expansion of frame 402 can occur due to a number of reasons. For example, native valve calcification can constrain the radial expansion of the frame 402 (and the corresponding prosthetic valve) at locations around the frame that contact calcified nodes formed on the native valve. In other words, if a portion of the frame comes into contact with a calcified node, that portion of the frame may not fully expand while the remaining portion of the frame may fully expand. Responsive to detecting the unevenness of the frame's radial expansion, a corrective action can be performed.

For example, the unevenly expanded frame 402 can be radially compressed (e.g., by operating the knob 212 of the delivery apparatus 200). In certain cases, the frame 402 only needs to be partially compressed as long as the frame 402 disengages from the calcified tissue. Then, the frame 402 can be repositioned axially and/or rotationally (e.g., via operating the knob 211 and/or other components of the delivery apparatus 200) so that the frame 402 is spaced away and/or oriented in a different direction relative to the calcified native tissue. Then, the frame 402 can be re-expanded (e.g., by operating the knob 212 of the delivery apparatus 200) while evenness of the frame's radial expansion can be continuously monitored, and such monitoring can be performed in real-time while radially expanding the frame 402.

As described herein, each of the first and second movable markers 440, 442 can be configured to be observable under fluoroscopy.

According to one example, each first movable marker 440 can be in the form of an aperture 444 extending through a thickness of the respective first actuator post 422, and each second movable marker 442 can include another aperture 446 extending through a thickness of the respective second actuator post 424. In the depicted example, both apertures 444 and 446 extend radially through a thickness of the respective first and second actuator posts 422, 424. In other examples, one or both apertures 444, 446 can extend in different directions, e.g., extending circumferentially through a thickness of the respective first and second actuator posts 422, 424.

According to another example, each first movable marker 440 can comprise a radiopaque material that has a different radiopacity than that of the respective first actuator post 422, and each second movable marker 442 can comprise a radiopaque material that has a different radiopacity than that of the respective second actuator post 424. In certain examples, the first and second movable markers 440, 442 can comprise the same radiopaque material. In other examples, the first and second movable markers 440, 442 can comprise different radiopaque materials. In certain examples, the radiopaque materials for the movable markers can be selected from any known or to be discovered radiopaque materials, such as iridium, tantalum, platinum, platinum-iridium alloy, Barium Sulphate, Bismuth Subcarbonate, Bismuth Oxychloride, or the like.

The radiopaque markers 440, 442 can have any of various shapes, such as disc-shaped, square, rectangular, oval, triangular, etc. The radiopaque markers 440, 442 can, for example, be mounted directly on the posts 422, 424, respectively, via various techniques or mechanisms, such as by welding the markers to the posts, an adhesive, mechanical fasteners, etc. In some examples, the markers 440, 442 can be disposed in correspondingly shaped apertures formed in the posts 422, 424 and fixed in place via a friction or press fit, welding, an adhesive, and/or mechanical fasteners.

According to yet another example, each first movable marker 440 can include a protrusion extending (e.g., radially inwardly, radially outwardly, circumferentially or sideways, etc.) from the respective first actuator post 422, and each second movable marker 442 can include another protrusion extending (e.g., radially inwardly, radially outwardly, circumferentially or sideways, etc.) from the respective second actuator post 424. The protrusions on the first and second actuator posts 422, 424 can extend in the same direction or different directions. Further, the protrusions on the first and second actuator posts 422, 424 can have the same or different sizes and/or shapes. The protrusions can enhance the visibility of the markers under fluoroscopy.

In certain examples, the types of the first and second movable markers 440, 442 can be mixed. For example, some of the first movable markers 440 (or second movable markers 442) can be configured as apertures while other first movable markers 440 (or second movable markers 442) can be configured as protrusions and/or markers attached to the posts. In another example, the first movable markers 440 can be configured as protrusions and the second movable markers 442 can be configured as apertures. In yet another example, at least some of the first and/or second movable markers 440, 442 can be configured as protrusions which comprise a radiopaque material that has different radiopacity than that of the respective first and/or second actuator posts 422, 424.

In certain examples, the two or more pairs of first movable markers 440 and second movable markers 442 can be uniformly distributed around a circumference of the frame 402. For example, if the frame 402 has six pairs of actuator posts 422, 424 that are uniformly distributed around the circumference of the frame, the first and second movable markers 440, 442 can be located on all six pairs of the actuator posts 422, 424, or on three equidistant pairs of the actuator posts 422, 424 that are 120 degrees from each other, or on two diametrically opposing pairs of the actuator posts 422, 424.

Alternatively, the two or more pairs of first movable markers 440 and second movable markers 442 can be nonuniformly distributed around the circumference of the frame 402. For example, if the frame 402 has six pairs of actuator posts 422, 424 that are uniformly distributed around the circumference of the frame, the first and second movable markers 440, 442 can be located on only two circumferentially adjacent pairs of the actuator posts 422, 424.

### Exemplary Techniques for Monitoring Frame Size During Radial Expansion

According to certain examples, the frame 402 can include at least one pair of first and second fixed markers 450, 452 that are axially spaced apart from one another by a fixed distance.

For example, as depicted in FIGS. 6-7, a pair of first and second fixed markers 450, 452 can be located on a selected support post 407. The first fixed marker 450 can be located on the support post 407 at a location that is adjacent to the first end 438 (or adjacent to the junction 411), and the second fixed marker 452 can be located on the support post 407 at a location that is adjacent to the second end 439. The axial distance between the first and second fixed markers 450, 452 (denoted as A0) can be a predefined constant value. In certain examples, A0 is slightly smaller than or about the same as the axial length (L2) of the support post 407. For example, the ratio A0/L2 can be between 50% and 100%, inclusive.

In certain examples, each support post 407 can have a corresponding pair of first and second fixed markers 450, 452. In other examples, only selected support posts 407 have the first and second fixed markers 450, 452. For example, if the frame 402 has six support posts 407, the first and second fixed markers 450, 452 can be located on any selected one, two, three, four, or five, or on all six support posts 407. When the frame 402 have multiple pairs of first and second fixed markers 450, 452, each pair of first and second fixed markers 450, 452 can have the same predefined axial distance A0 therebetween.

As described herein, each of the first and second fixed markers 450, 452 can be configured to be observable under fluoroscopy.

According to one example, the first and second fixed markers 450, 452 can include respective apertures 456 extending through a thickness of the corresponding support post 407. In the depicted example, both apertures 456 extend radially through a thickness of the support post 407. In other examples, one or both apertures 456 can extend in different directions, e.g., extending circumferentially through a thickness of the support post 407.

According to another example, the first and second fixed markers 450, 452 can include a radiopaque material that has a different radiopacity than that of corresponding support post 407. In certain examples, the first and second fixed markers 450, 452 can comprise the same radiopaque material. In other examples, the first and second fixed markers 450, 452 can comprise different radiopaque materials. In certain examples, the radiopaque materials for the fixed markers can be selected from any known or to be discovered radiopaque materials, such as iridium, tantalum, platinum, platinum-iridium alloy, Barium Sulphate, Bismuth Subcarbonate, Bismuth Oxychloride, or the like.

The radiopaque markers 450, 452 can have any of various shapes, such as disc-shaped, square, rectangular, oval, triangular, etc. The radiopaque markers 450, 452 can, for example, be mounted directly on the posts 407, via various techniques or mechanisms, such as by welding the markers to the posts, an adhesive, mechanical fasteners, etc. In some examples, the markers 450, 452 can be disposed in correspondingly shaped apertures formed in the posts 407 and fixed in place via a friction or press fit, welding, an adhesive, and/or mechanical fasteners.

According to yet another example, the first and second fixed markers 450, 452 can include respective protrusions extending (e.g., radially inwardly, radially outwardly, circumferentially or sideways, etc.) from the corresponding support post 407. The protrusions on the support posts 407 can extend in the same direction or different directions. Further, the protrusions on the support posts 407 can have the same or different sizes and/or shapes.

In certain examples, the types of the first and second fixed markers 452, 454 can be mixed. For example, some of the fixed markers 452 and/or 454 can be configured as apertures while other fixed markers 452 and/or 454 can be configured as protrusions and/or markers attached to the posts 407. In another example, at least some of the fixed markers 452 and/or 454 can be configured as protrusions which comprise a radiopaque material that has different radiopacity than that of the corresponding support posts 407.

In certain examples, multiple pairs of first and second fixed markers 450, 452 can be uniformly distributed around a circumference of the frame 402. For example, if the frame 402 has six support posts 407 that are uniformly distributed around the circumference of the frame, the pairs of first and second fixed markers 450, 452 can be located on all six support posts 407, or on three equidistant support posts 407 that are 120 degrees from each other, or on two diametrically opposing support posts 407. Alternatively, multiple pairs of first and second fixed markers 450, 452 can be nonuniformly distributed around the circumference of the frame 402.

According to certain examples, the predefined axial distance A0 can serve as a reference or calibration value, based on which the axial length and/or the radial diameter of the frame 402 can be estimated, thus allowing real-time monitoring the size of the frame 402 during its radial expansion and/or compression.

For example, the axial distance between a selected pair of first and second movable markers (e.g., A1, A2, etc.) can be compared with the constant axial distance A0. Such comparation can be performed visually, e.g., under the fluoroscopy, by an operator (e.g., a physician). Based on such comparison, the axial distance between the selected pair of first and second movable markers can be determined or estimated based on A0. In certain examples, one or more calibration curves (or tables) can be provided which characterize the relationship between the axial distance between the selected pair of first and second movable markers and the axial length and/or radial diameter of the frame 402. Thus, the axial length and/or radial diameter of the frame 402 can also be determined or estimated by comparing the axial distance between the selected pair of first and second movable markers with the predefined axial distance A0.

Moreover, each pair of moveable markers 440, 442 can be compared to a pair of fixed markers 450, 452 to determine if the frame is expanding in an acceptable manner with the required degree of uniform expansion. For example, if the relative distances between A1 and A0 is greater than or less than the relative distances between A2 and A0, this can indicate that the cell columns or frame portions corresponding to A1 and A2 are expanding unevenly. If the difference in frame expansion between two cell columns or frame portions exceeds a predetermined value or is otherwise unacceptable, then the user may take corrective action by at least partially radially compressing the prosthetic valve, re-positioning the prosthetic valve, and then re-expanding the prosthetic valve.

### Sterilization

Any of the systems, devices, apparatuses, etc. herein can be sterilized (for example, with heat/thermal, pressure, steam, radiation, and/or chemicals, etc.) to ensure they are safe for use with patients, and any of the methods herein can include sterilization of the associated system, device, apparatus, etc. as one of the steps of the method. Examples of heat/thermal sterilization include steam sterilization and autoclaving. Examples of radiation for use in sterilization include, without limitation, gamma radiation, ultra-violet radiation, and electron beam. Examples of chemicals for use in sterilization include, without limitation, ethylene oxide, hydrogen peroxide, peracetic acid, formaldehyde, and glutaraldehyde. Sterilization with hydrogen peroxide may be accomplished using hydrogen peroxide plasma, for example.

The features described herein with regard to any example can be combined with other features described in any one or more of the other examples, unless otherwise stated. For example, any one or more of the features of one prosthetic device/valve can be combined with any one or more features of another prosthetic device/valve.

In view of the many possible examples to which the principles of the disclosed technology may be applied, it should be recognized that the illustrated examples are only preferred examples of the technology and should not be taken as limiting the scope of the disclosure. Rather, the scope of the claimed subject matter is defined by the following claims.

## Claims

1. A prosthetic heart valve (100), comprising:
a radially compressible and expandable frame (102; 402);
wherein the frame (102; 402) comprises a pair of first (450) and second (452) fixed markers that are axially spaced apart from one another by a fixed distance and a pair of first (440) and second (442) movable markers that are axially movable relative to one another as the frame radially expands so that a distance between the pair of first (440) and second (442) movable markers can be compared to the fixed distance between the pair of first (450) and second (452) fixed markers.

2. The prosthetic heart valve (100) of claim 1, wherein the pair of first (450) and second (452) fixed markers are located on an axially extending support post (107; 407) of the frame (102; 402).

3. The prosthetic heart valve (100) of claim 2, wherein each of the first (450) and second (452) fixed markers comprises an aperture (456) extending through a thickness of the support post (107; 407).

4. The prosthetic heart valve (100) of any one of claims 2 to 3, wherein the first (450) and second (452) fixed markers comprise a radiopaque material that has a different radiopacity than that of the support post (107; 407).

5. The prosthetic heart valve (100) of any one of claims 2 to 4, wherein the support post (107; 407) is one of a plurality of support posts (107; 407), each one of the plurality of support posts (107; 407) comprising a respective pair of the first (450) and second (452) fixed markers having the fixed distance therebetween.

6. The prosthetic heart valve (100) of any one of claims 1 to 5, wherein the frame (102; 402) is mechanically expandable and the valve further comprises a screw actuator (126; 426) coupled to the frame (102; 402) and configured to be rotated to radially expand the frame (102; 402) from a radially compressed state to a radially expanded state.

7. The prosthetic heart valve (100) of claim 6, wherein the frame (102; 402) comprises a plurality of pairs of first (122; 422) and second (124; 424) posts, wherein the first (122; 422) and second (124; 424) posts of each pair are axially movable relative to one another, wherein the screw actuator (126; 426) extends through an inner bore of the second post (124; 424) and is coupled to the first post (122: 422) of a selected pair of posts, wherein the first post (122; 422) of the selected pair comprises the first movable marker (440) and the second post (124; 424) of the selected pair comprises the second movable marker (442).

8. The prosthetic heart valve (100) of claim 7, wherein the first movable marker (440) comprises a first aperture (444) extending through the first post (122; 422) of the selected pair, and the second movable marker (442) comprises a second aperture (446) extending through the second post (124; 424) of the selected pair.

9. The prosthetic heart valve (100) of any one of claims 7 to 8, wherein the first (440) and second (442) movable markers comprise a radiopaque material that has a different radiopacity than that of the first (122; 422) and second (124; 424) posts.

10. The prosthetic heart valve (100) of any one of claims 7 to 9, wherein the screw actuator (126; 426) is one of a plurality of screw actuators (126; 426), each screw actuator (126; 426) extending through a respective pair of first (122; 422) and second (124; 424) posts, wherein the plurality of screw actuators (126; 426) are configured to be rotated in a first direction to radially expand the frame (102; 402) and rotated in an opposite second direction to radially compress the frame (102; 402).

11. The prosthetic heart valve (100) of any one of claims 7 to 10, wherein the frame (102; 402) further comprises a plurality of axially extending third posts, wherein each third post is positioned between two pairs of first (122; 422) and second (124; 424) posts and each pair of first (122; 422) and second (124; 424) posts are positioned between two third posts, wherein a plurality of struts (112; 412) extending between and connecting the plurality of pairs of first (122; 422) and second (124; 424) posts and the plurality of third posts define a plurality of cells (117; 118; 417; 418) that extend circumferentially around the frame.

12. The prosthetic heart valve (100) of any one of claims 1 to 11, comprising two or more pairs of first movable markers (440) and second movable markers (442), wherein the two or more pairs of first movable markers (440) and second movable markers (442) are uniformly distributed around a circumference of the frame (102; 402).

13. The prosthetic heart valve (100) of any one of claims 1 to 12, wherein the frame (102; 402) comprises six pairs of first movable markers (440) and second movable markers (442).

14. The prosthetic heart valve (100) of any one of claims 12 and 13, wherein positions of the first (440) and second (442) movable markers are configured so that a difference between an axial distance of one of the pairs of first (440) and second (442) movable markers and an axial distance of another one of the pairs of first (440) and second (442) movable markers can indicate a degree of evenness of the radial expansion of the frame (102; 402).

15. The prosthetic heart valve (100) of any one of claims 1 to 14, further comprising a leaflet structure (150) including a plurality of leaflets (158) and an outer skirt (103).

## Patentansprüche

1. Herzklappenprothese (100), umfassend:
einen radial komprimierbaren und expandierbaren Rahmen (102; 402);
wobei der Rahmen (102; 402) ein Paar aus erster (450) und zweiter (452) fester Markierung, die axial voneinander durch einen festen Abstand beabstandet sind, und ein Paar aus erster (440) und zweiter (442) beweglicher Markierung umfasst, die axial relativ zueinander beweglich sind, wenn der Rahmen radial expandiert, so dass ein Abstand zwischen dem Paar der ersten (440) und der zweiten (442) beweglichen Markierung mit dem festen Abstand zwischen dem Paar der ersten (450) und der zweiten (452) festen Markierungen verglichen werden kann.

2. Herzklappenprothese (100) nach Anspruch 1, wobei das Paar der ersten (450) und der zweiten (452) festen Markierung an einem sich axial erstreckenden Stützpfosten (107; 407) des Rahmens (102; 402) angeordnet ist.

3. Herzklappenprothese (100) nach Anspruch 2, wobei die erste (450) und die zweite (452) feste Markierung jeweils eine Öffnung (456) aufweisen, die sich durch eine Dicke des Stützpfostens (107; 407) erstreckt.

4. Herzklappenprothese (100) nach einem der Ansprüche 2 bis 3, wobei die erste (450) und die zweite (452) feste Markierung ein röntgendichtes Material umfassen, das eine andere Röntgensichtbarkeit aufweist als der Stützpfosten (107; 407).

5. Herzklappenprothese (100) nach einem der Ansprüche 2 bis 4, wobei der Stützpfosten (107; 407) einer von mehreren Stützpfosten (107; 407) ist, wobei jeder der mehreren Stützpfosten (107; 407) ein jeweiliges Paar der ersten (450) und der zweiten (452) festen Markierung mit dem festen Abstand dazwischen umfasst.

6. Herzklappenprothese (100) nach einem der Ansprüche 1 bis 5, wobei der Rahmen (102; 402) mechanisch expandierbar ist und die Klappe ferner einen Schraubenaktuator (126; 426) umfasst, der mit dem Rahmen (102; 402) gekoppelt ist und dazu konfiguriert ist, gedreht zu werden, um den Rahmen (102; 402) von einem radial komprimierten Zustand in einen radial expandierten Zustand zu expandieren.

7. Herzklappenprothese (100) nach Anspruch 6, wobei der Rahmen (102; 402) mehrere Paare von ersten (122; 422) und zweiten (124; 424) Pfosten umfasst, wobei die ersten (122; 422) und die zweiten (124; 424) Pfosten jedes Paares relativ zueinander axial beweglich sind, wobei sich der Schraubenaktuator (126; 426) durch eine Innenbohrung des zweiten Pfostens (124; 424) erstreckt und mit dem ersten Pfosten (122; 422) eines ausgewählten Pfostenpaares gekoppelt ist, wobei der erste Pfosten (122; 422) des ausgewählten Paares die erste bewegliche Markierung (440) umfasst und der zweite Pfosten (124; 424) des ausgewählten Paares die zweite bewegliche Markierung umfasst.

8. Herzklappenprothese (100) nach Anspruch 7, wobei die erste bewegliche Markierung (440) eine erste Öffnung (444) umfasst, die sich durch den ersten Pfosten (122; 422) des ausgewählten Paares erstreckt, und die zweite bewegliche Markierung (442) eine zweite Öffnung (446) umfasst, die sich durch den zweiten Pfosten (124; 424) des ausgewählten Paares erstreckt.

9. Herzklappenprothese (100) nach einem der Ansprüche 7 bis 8, wobei die erste (440) und die zweite (442) bewegliche Markierung ein röntgendichtes Material umfassen, das eine andere Röntgensichtbarkeit aufweist als der erste (122; 422) und der zweite (124; 424) Pfosten.

10. Herzklappenprothese (100) nach einem der Ansprüche 7 bis 9, wobei der Schraubenaktuator (126; 426) einer von mehreren Schraubenaktuatoren (126; 426) ist, wobei sich jeder Schraubenaktuator (126; 426) durch ein jeweiliges Paar von erstem (122; 422) und zweitem (124; 424) Pfosten erstreckt, wobei die mehreren Schraubenaktuatoren (126; 426) dazu konfiguriert sind, in einer ersten Richtung gedreht zu werden, um den Rahmen (102; 402) radial zu expandieren, und in einer entgegengesetzten zweiten Richtung gedreht zu werden, um den Rahmen (102; 402) radial zu komprimieren.

11. Herzklappenprothese (100) nach einem der Ansprüche 7 bis 10, wobei der Rahmen (102; 402) ferner mehrere sich axial erstreckende dritte Pfosten aufweist, wobei jeder dritte Pfosten zwischen zwei Paaren von ersten (122; 422) und zweiten (124; 424) Pfosten angeordnet ist und jedes Paar von ersten (122; 422) und zweiten (124; 424) Pfosten zwischen zwei dritten Pfosten angeordnet ist, wobei mehrere Streben (112; 412), die sich zwischen den mehreren Paaren von ersten (122; 422) und zweiten (124; 424) Pfosten und den mehreren dritten Pfosten erstrecken und diese verbinden, mehrere Zellen (117; 118; 417; 418) definieren, die sich in Umfangsrichtung um den Rahmen herum erstrecken.

12. Herzklappenprothese (100) nach einem der Ansprüche 1 bis 11, die zwei oder mehr Paare von ersten beweglichen Markierungen (440) und zweiten beweglichen Markierungen (442) umfasst, wobei die zwei oder mehr Paare von ersten beweglichen Markierungen (440) und zweiten beweglichen Markierungen (442) gleichmäßig um einen Umfang des Rahmens (102; 402) verteilt sind.

13. Herzklappenprothese (100) nach einem der Ansprüche 1 bis 12, wobei der Rahmen (102; 402) sechs Paare von ersten beweglichen Markierungen (440) und zweiten beweglichen Markierungen (442) umfasst.

14. Herzklappenprothese (100) nach einem der Ansprüche 12 und 13, wobei die Positionen der ersten (440) und zweiten (442) beweglichen Markierungen so konfiguriert sind, dass eine Differenz zwischen einem axialen Abstand eines der Paare der ersten (440) und zweiten (442) beweglichen Markierungen und einem axialen Abstand eines weiteren der Paare der ersten (440) und zweiten (442) beweglichen Markierungen einen Grad der Gleichmäßigkeit der radialen Expansion des Rahmens (102; 402) anzeigen kann.

15. Herzklappenprothese (100) nach einem der Ansprüche 1 bis 14, die ferner eine Segelstruktur (150) umfasst, die mehrere Segel (158) und eine äußere Schürze (103) aufweist.

## Revendications

1. Valvule cardiaque prothétique (100) comprenant :
un cadre radialement compressible et déployable (102 ; 402) ;
le cadre (102 ; 402) comprenant une paire de premier (450) et second (452) marqueurs fixes qui sont écartés axialement l'un de l'autre d'une distance fixe et une paire de premier (440) et second (442) marqueurs mobiles qui sont axialement mobiles l'un par rapport à l'autre à mesure que le cadre se déploie radialement de telle sorte qu'une distance entre la paire de premier (440) et second (442) marqueurs mobiles peut être comparée à la distance fixe entre la paire de premier (450) et second (452) marqueurs fixes.

2. Valvule cardiaque prothétique (100) selon la revendication 1, la paire de premier (450) et second (452) marqueurs fixes étant située sur un montant support (107 ; 407) s'étendant axialement du cadre (102 ; 402).

3. Valvule cardiaque prothétique (100) selon la revendication 2, chacun des premier (450) et second (452) marqueurs fixes comprenant un orifice (456) s'étendant à travers une épaisseur du montant support (107 ; 407).

4. Valvule cardiaque prothétique (100) selon l'une quelconque des revendications 2 à 3, les premier (450) et second (452) marqueurs fixes comprenant un matériau radio-opaque qui présente une radio-opacité différente de celle du montant support (107 ; 407).

5. Valvule cardiaque prothétique (100) selon l'une quelconque des revendications 2 à 4, le montant support (107 ; 407) étant un montant parmi une pluralité de montants support (107 ; 407), chaque montant parmi la pluralité de montants support (107 ; 407) comprenant une paire respective des premier (450) et second (452) marqueurs fixes présentant la distance fixe entre eux.

6. Valvule cardiaque prothétique (100) selon l'une quelconque des revendications 1 à 5, le cadre (102 ; 402) étant déployable mécaniquement et la valvule comprenant en outre un actionneur à vis (126 ; 426) accouplé au cadre (102 ; 402) et conçu pour être tourné afin de déployer radialement le cadre (102 ; 402) à partir d'un état radialement comprimé vers un état radialement déployé.

7. Valvule cardiaque prothétique (100) selon la revendication 6, le cadre (102 ; 402) comprenant une pluralité de paires de premier (122 ; 422) et deuxième (124 ; 424) montants, les premier (122 ; 422) et deuxième (124 ; 424) montants de chaque paire étant axialement mobiles l'un par rapport à l'autre, l'actionneur à vis (126 ; 426) s'étendant à travers un trou interne du deuxième montant (124 ; 424) et étant accouplé au premier montant (122 : 422) d'une paire sélectionnée de montants, le premier montant (122 ; 422) de la paire sélectionnée comprenant le premier marqueur mobile (440) et le deuxième montant (124 ; 424) de la paire sélectionnée comprenant le second marqueur mobile (442).

8. Valvule cardiaque prothétique (100) selon la revendication 7, le premier marqueur mobile (440) comprenant un premier orifice (444) s'étendant à travers le premier montant (122 ; 422) de la paire sélectionnée et le second marqueur mobile (442) comprenant un second orifice (446) s'étendant à travers le deuxième montant (124 ; 424) de la paire sélectionnée.

9. Valvule cardiaque prothétique (100) selon l'une quelconque des revendications 7 à 8, les premier (440) et second (442) marqueurs mobiles comprenant un matériau radio-opaque qui présente une radio-opacité différente de celle des premier (122 ; 422) et deuxième (124 ; 424) montants.

10. Valvule cardiaque prothétique (100) selon l'une quelconque des revendications 7 à 9, l'actionneur à vis (126 ; 426) étant un actionneur parmi une pluralité d'actionneurs à vis (126 ; 426), chaque actionneur à vis (126 ; 426) s'étendant à travers une paire respective de premier (122 ; 422) et deuxième (124 ; 424) montants, la pluralité d'actionneurs à vis (126 ; 426) étant conçus pour être tournés dans un premier sens afin de déployer radialement le cadre (102 ; 402) et pour être tournés dans un second sens opposé afin de comprimer radialement le cadre (102 ; 402).

11. Valvule cardiaque prothétique (100) selon l'une quelconque des revendications 7 à 10, le cadre (102 ; 402) comprenant en outre une pluralité de troisièmes montants s'étendant axialement, chaque troisième montant étant positionné entre deux paires de premier (122 ; 422) et deuxième (124 ; 424) montants et chaque paire de premier (122 ; 422) et deuxième (124 ; 424) montants étant positionnée entre deux troisièmes montants, une pluralité d'entretoises (112 ; 412) s'étendant entre la pluralité de paires de premier (122 ; 422) et deuxième (124 ; 424) montants et les reliant et la pluralité de troisièmes montants définissant une pluralité de cellules (117 ; 118 ; 417 ; 418) qui s'étendent circonférentiellement autour du cadre.

12. Valvule cardiaque prothétique (100) selon l'une quelconque des revendications 1 à 11, comprenant deux paires ou plus de premiers marqueurs mobiles (440) et de seconds marqueurs mobiles (442), les deux paires ou plus de premiers marqueurs mobiles (440) et de seconds marqueurs mobiles (442) étant uniformément réparties autour d'une circonférence du cadre (102 ; 402).

13. Valvule cardiaque prothétique (100) selon l'une quelconque des revendications 1 à 12, le cadre (102 ; 402) comprenant six paires de premiers marqueurs mobiles (440) et de seconds marqueurs mobiles (442).

14. Valvule cardiaque prothétique (100) selon l'une quelconque des revendications 12 et 13, les positions des premier (440) et second (442) marqueurs mobiles étant conçues de telle sorte qu'une différence entre une distance axiale entre l'une des paires de premier (440) et second (442) marqueurs mobiles et une distance axiale entre une autre des paires de premier (440) et second (442) marqueurs mobiles peut indiquer un degré d'uniformité du déploiement radial du cadre (102 ; 402).

15. Valvule cardiaque prothétique (100) selon l'une quelconque des revendications 1 à 14, comprenant en outre une structure (150) à feuillets comprenant une pluralité de feuillets (158) et une collerette externe (103).
